(19) Europäisches Patentamt
European Patent Office
Office européen des brevets

(11) Veröffentlichungsnummer: **0 528 242 A2**

(12) # EUROPÄISCHE PATENTANMELDUNG

(21) Anmeldenummer: **92113173.6**

(22) Anmeldetag: **03.08.92**

(51) Int. Cl.5: **C07K 5/06**, C07K 5/08, C07D 207/08, C07D 211/18, C07D 215/12, C07D 217/14, A61K 31/40, A61K 31/445, A61K 31/47, A61K 37/64

(30) Priorität: **10.08.91 DE 4126485**

(43) Veröffentlichungstag der Anmeldung: **24.02.93 Patentblatt 93/08**

(84) Benannte Vertragsstaaten: **AT BE CH DE DK ES FR GB GR IE IT LI LU MC NL PT SE**

(71) Anmelder: **BAYER AG**
**W-5090 Leverkusen 1 Bayerwerk(DE)**

(72) Erfinder: **Häbisch, Dieter, Dr.**
**Krummacher Strasse 82**
**W-5600 Wuppertal 1(DE)**
Erfinder: **Röben, Wolfgang, Dr.**
**Strässchen Siefen 30**
**W-5060 Bergisch Gladbach 2(DE)**
Erfinder: **Hansen, Jutta, Dr.**
**Clauduisweg 9**
**W-5600 Wuppertal 1(DE)**
Erfinder: **Paessens, Arnold, Dr.**
**Stresemannstrasse 51**
**W-5657 Haan(DE)**

(54) **Trifluormethyl-haltige Pseudopeptide.**

(57) Die Erfindung betrifft Trifluormethyl-haltige Pseudopeptide der allgemeinen Formel (I)

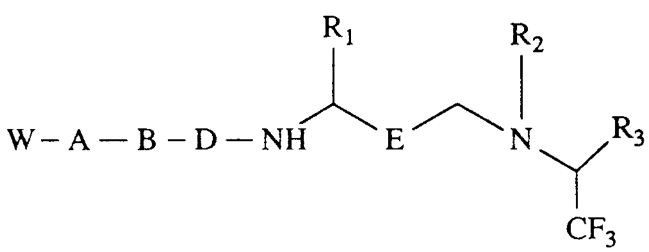

(I)

in welcher W, A, B, D, E, $R^1$, $R^2$ und $R^3$ die in der Beschreibung angegebene Bedeutung haben, Verfahren zu ihrer Herstellung und ihre Verwendung als retrovirale Mittel.

EP 0 528 242 A2

Die Erfindung betrifft Trifluormethyl-haltige Pseudopeptide, Verfahren zu ihrer Herstellung und ihre Verwendung als anti-retrovirale Mittel.

Es wurde bereits versucht, Peptide und Pseudopeptide, die teilweise auch renininhibitorisch wirksam sind, bei der Bekämpfung von AIDS einzusetzen [vgl. WO 90/09191; WO 90/12804; EP 393 445; EP 402 646; EP 373 576].

Die vorliegende Erfindung betrifft neue Trifluormethyl-haltige Pseudopeptide der allgemeinen Formel (I)

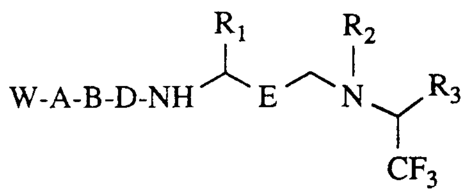 (I)

in welcher

W für Wasserstoff oder für eine typische Aminoschutzgruppe steht, oder für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind oder für eine Gruppe der Formel $R^4$-CO-, $R^5R^6$N-CO- oder $R^7$-$SO_2$- steht,

worin

$R^4$ Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach gleich oder verschieden durch Aryl mit 6 bis 10 Kohlenstoffatomen oder Pyridyl substituiert ist, oder

Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,

Cycloalkyl mit 3 bis 7 Kohlenstoffatomen bedeutet, oder

Chinolyl, Chinolyl-N-oxid, Indolyl, Pyridyl, Morpholino oder

Piperazinyl bedeutet, oder

einen Rest der Formel

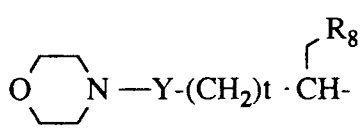 , 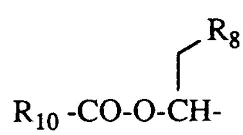 ,

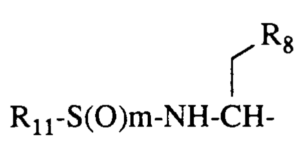 , 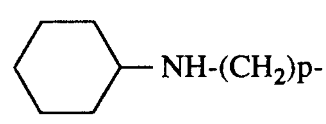 ,

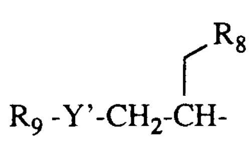 oder 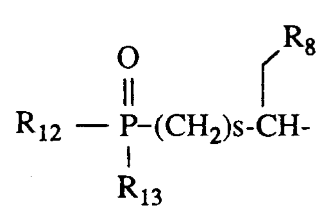

bedeutet,

worin

$R^8$ Phenyl oder Naphthyl bedeutet,

$R^9$ und $R^{10}$ unabhängig voneinander geradkettiges oder verzweigtes Alkyl mit bis zu 14 Kohlenstoffatomen bedeuten, das gegebenenfalls durch Phenyl oder Naphthyl substituiert ist, oder Benzyloxy oder Aryl mit 6 bis 10 Kohlenstoffatomen bedeuten, welches seinerseits durch Alkyl mit bis zu 4 Kohlenstoffatomen substituiert ist,

$R^{11}$ die oben angegebene Bedeutung von $R^9$ und $R^{10}$ hat und mit dieser gleich oder verschieden ist oder über N-gebundenes Morpholino oder Pyrrolidinyl bedeutet,

m eine Zahl 0, 1 oder 2 bedeutet,

p eine Zahl 1, 2 oder 3 bedeutet,

Y und Y' unabhängig voneinander für CO- oder $SO_2$- stehen,

t eine Zahl 0, 1 oder 2 bedeutet,

$R^{12}$ und $R^{13}$ unabhängig voneinander Hydroxy oder Alkoxy mit bis zu 8 Kohlenstoffatomen bedeuten,

s eine Zahl 1 oder 2 bedeutet,

$R^5$ und $R^6$ unabhängig voneinander Wasserstoff oder
Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert sein kann, oder
Cycloalkyl mit 3 bis 7 Kohlenstoffatomen,
oder geradkettiges oder verzweigtes Alkyl mit bis zu 18 Kohlenstoffatomen bedeuten, das gegebenenfalls durch Pyridyl substituiert ist,

$R^7$ geradkettiges oder verzweigtes Alkyl mit bis zu 8 Kohlenstoffatomen bedeutet,

A, B und D gleich oder verschieden sind und
für eine direkte Bindung oder
für einen Rest der Formel

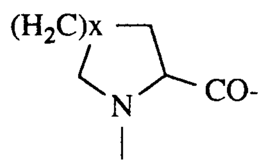 oder 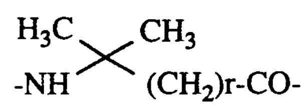

stehen ,
worin

x die Zahl 1 oder 2 bedeutet
und

r die Zahl 0 oder 1 bedeutet,
oder
für eine Gruppe der Formel

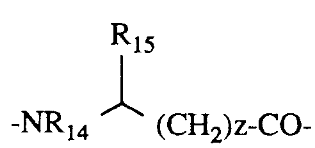

stehen
worin

z die Zahl 0 oder 1 bedeutet,

$R^{14}$ Wasserstoff oder geradkettiges oder verzweigtes Alkyl mit bis zu 4 Kohlenstoffatomen bedeutet,

$R^{15}$ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,

wobei das Alkyl gegebenenfalls durch Cyano, Methylthio, Hydroxy, Mercapto, Guanidyl oder durch eine Gruppe der Formel $-NR^{16}R^{17}$ oder $R^{18}$-OC- substituiert ist,
worin

$R^{16}$ und $R^{17}$ unabhängig voneinander für Wasserstoff, geradkettiges oder verzweigtes Alkyl mit bis zu 8 Kohlenstoffatomen oder Phenyl stehen,

und

$R^{18}$ Hydroxy, Benzyloxy, Alkoxy mit bis zu 6 Kohlenstoffatomen oder die oben aufgeführte Gruppe $-NR^{16}R^{17}$ bedeutet, oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^{16}R^{17}$ substituiert ist,

worin

$R^{16}$ und $R^{17}$ die oben angegebene Bedeutung haben,

oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^1$ für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

E für einen Rest der Formel

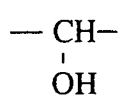

oder

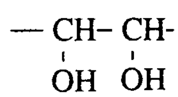

steht,

$R^2$ und $R^3$ gleich oder verschieden sind und

für Wasserstoff oder für geradkettiges oder verzweigtes Alkyl mit bis zu 8 Kohlenstoffatomen stehen, oder

$R^2$ und $R^3$ gemeinsam mit dem Stickstoffatom und unter Einbezug der

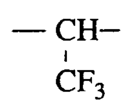

-Gruppe einen 5- bis 7-gliedrigen gesättigten Heterocyclus bilden, an den ein weiterer 5- bis 6-gliedriger, gesättigter, partiell ungesättigter oder aromatischer Carbocyclus ankondensiert sein kann, wobei beide Ringe gegebenenfalls durch geradkettiges oder verzweigtes Alkyl mit bis zu 8 Kohlenstoffatomen substituiert sind

und deren physiologisch unbedenklichen Salze.

Die erfindungsgemäßen Verbindungen der allgemeinen Formel (I), haben mehrere asymmetrische Kohlenstoffatome. Sie können unabhängig voneinander in der D-oder L-Form vorliegen. Die Erfindung umfaßt die optischen Antipoden ebenso wie die Isomerengemische oder Racemate. Bevorzugt liegen die Gruppen A, B und D unabhängig voneinander in der optisch reinen, bevorzugt in der L-Form vor.

Der Rest der allgemeinen Formel (II)

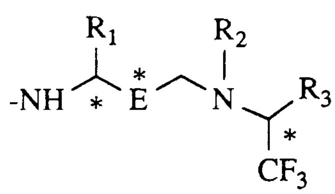

(II)

besitzt zunächst in Abhängigkeit von der Bedeutung des Restes E mindestens 3 oder 4 Kohlenstoffatome (*), wobei im Fall, daß $R^2$ und $R^3$ unter Einbezug des Stickstoffatoms ein heterocyclisches, gegebenenfalls substituiertes Ringssystem bilden, die Anzahl der Asymmetriezentren zunimmt. Die asymmetrischen Kohlenstoffatome können unabhängig voneinander in der R- oder S-Konfiguration vorliegen.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloraceryl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Die erfindungsgemäßen Verbindungen der allgemeinen Formel (I) können in Form ihrer Salze vorliegen. Dies können Salze mit anorganischen oder organischen Säuren oder Basen sein.

Bevorzugt sind Verbindungen der allgemeinen Formel (I),

in welcher

W für Wasserstoff, tert.Butyloxycarbonyl (BOC), 9-Fluorenylmethyloxycarbonyl (Fmoc), Benzyloxycarbonyl (Z) oder Pyridylmethoxycarbonyl steht, oder
für geradkettiges oder verzweigtes Alkyl oder Alkenyl mit jeweils bis zu 4 Kohlenstoffatomen steht, die gegebenenfalls durch Phenyl substituiert sind, oder
für eine Gruppe der Formel $R^4$-CO-, $R^5R^6N$-CO- oder $R^7SO_2$- steht
worin

$R^4$ Wasserstoff, Trifluormethyl oder geradkettiges oder verzweigtes Alkoxy mit bis zu 6 Kohlenstoffatomen oder Alkyl mit bis zu 16 Kohlenstoffatomen bedeutet, das gegebenenfalls bis zu 2-fach, gleich oder verschieden, durch Phenyl, Naphthyl oder Pyridyl substituiert sein kann, oder
Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sein können,
Cyclopropyl, Cyclopentyl, Cyclohexyl, Chinolyl, Chinolyl-N-oxid, Indolyl, Pyridyl, Morpholino oder Piperazinyl bedeutet, oder
einen Rest der Formel

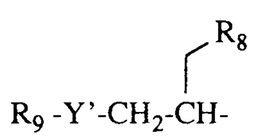

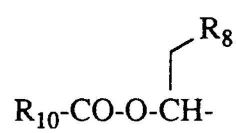

oder

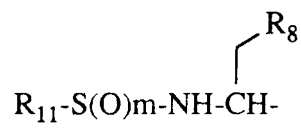

bedeutet,
worin

Y' die CO- oder $SO_2$-Gruppe bedeutet,

$R^8$ Phenyl oder Naphthyl bedeutet,

$R^9$ und $R^{10}$ unabhängig voneinander geradkettiges oder verzweigtes Alkyl mit bis zu 8 Kohlenstoffatomen, Tolyl, Benzyloxy, Phenyl oder Naphthyl bedeuten,

$R^{11}$ die oben angegebene Bedeutung von $R^9$ und $R^{10}$ hat und mit dieser gleich oder verschieden ist oder über N-gebundenes Morpholino oder Pyrrolidinyl bedeutet,

m eine Zahl 1 oder 2 bedeutet,

$R^5$ und $R^6$ unabhängig voneinander Wasserstoff oder Phenyl oder Naphthyl bedeuten, die gegebenenfalls durch geradkettiges oder verzweigtes Alkyl mit bis zu 4 Kohlenstoffatomen, Fluor oder Chlor substituiert sein können, Cyclopropyl, Cyclopentyl oder Cyclohexyl bedeuten, oder geradkettiges oder verzweigtes Alkyl mit bis zu 16 Kohlenstoffatomen bedeuten, das gegebenenfalls durch Pyridyl substituiert ist,

$R^7$ geradkettiges oder verzweigtes Alkyl mit bis zu 6 Kohlenstoffatomen bedeutet,

A, B und D unabhängig voneinander für eine direkte Bindung oder für Prolin stehen, oder für einen Rest der Formel

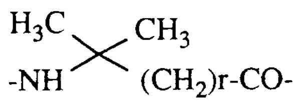

stehen,
worin

r die Zahl 0 oder 1 bedeutet
für eine Gruppe der Formel

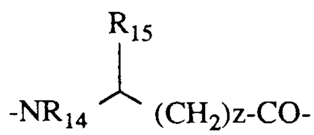

stehen,
worin

z die Zahl 0 oder 1 bedeutet,

$R^{14}$ Wasserstoff, Methyl oder Ethyl bedeutet,

$R^{15}$ Cyclopentyl, Cyclohexyl, Phenyl oder Wasserstoff bedeutet, oder geradkettiges oder verzweigtes Alkyl mit bis zu 6 Kohlenstoffatomen bedeutet,
wobei das Alkyl gegebenenfalls durch Cyano, Methylthio, Hydroxy, Mercapto, Guanidyl, Amino, Carboxy oder $H_2N$-CO- substituiert sein kann,
oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,
oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^1$ für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert sein kann

E für einen Rest der Formel

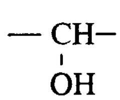

oder

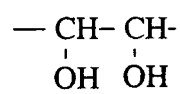

steht,

$R^2$ und $R^3$ gleich oder verschieden sind und für Wasserstoff oder für geradkettiges oder verzweigtes Alkyl mit bis zu 6 Kohlenstoffatomen stehen, oder

$R^2$ und $R^3$ gemeinsam mit dem Stickstoffatom und unter Einbezug der

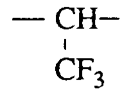

-Gruppe für einen Rest der Formel

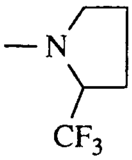 , 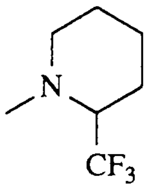 , 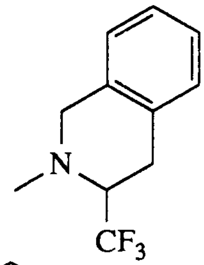 , 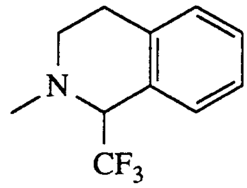 ,

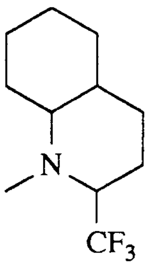 , 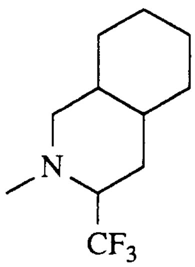 , 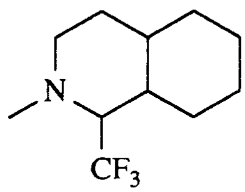

oder 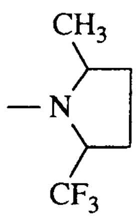

wobei beide Ringe gegebenenfalls durch geradkettiges oder verzweigtes Alkyl mit bis zu 6 Kohlenstoffatomen substituiert sind

und deren physiologisch unbedenklichen Salze.

Besonders bevorzugt sind Verbindungen der allgemeinen Formel (I),

in welcher

W für Wasserstoff, tert.Butyloxycarbonyl (BOC), Benzyloxycarbonyl (Z) oder Pyridylmethoxycarbonyl steht, oder
für Allyl oder Benzyl steht,
für eine Gruppe der Formel $R^4$-CO-, $R^5R^6$N-CO- oder $R^7$-$SO_2$- steht,
worin

$R^4$ Wasserstoff oder geradkettiges oder verzweigtes Alkoxy mit bis zu 4 Kohlenstoffatomen, Alkyl mit bis zu 14 Kohlenstoffatomen bedeutet, das gegebenenfalls bis zu 2-fach durch Phenyl, Naphthyl oder Pyridyl substituiert sein kann, oder
Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert sein kann,
Cyclopropyl, Cyclopentyl, Cyclohexyl, Chinolyl, Chinolyl-N-oxid, Indolyl, Pyridyl, Morpholino oder Piperazinyl bedeutet, oder einen Rest der Formel

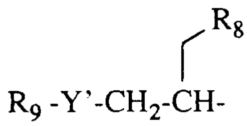 oder 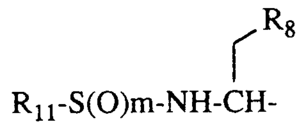

bedeutet,
worin

| | |
|---|---|
| Y' | die CO- oder $SO_2$-Gruppe bedeutet, |
| $R^8$ | Phenyl oder Naphthyl bedeutet |
| $R^9$ | geradkettiges oder verzweigtes Alkyl mit bis zu 4 Kohlenstoffatomen, Tolyl, Benzyloxy, Phenyl oder Naphthyl bedeutet, |
| $R^{11}$ | die oben angegebene Bedeutung von $R^9$ hat und mit dieser gleich oder verschieden ist oder über N-gebundenes Morpholino oder Pyrrolidinyl bedeutet, |
| m | die Zahl 2 bedeutet, |
| $R^5$ und $R^6$ | unabhängig voneinander Wasserstoff oder Phenyl oder Naphthyl bedeuten, die gegebenenfalls durch Methyl, Fluor oder Chlor substituiert sind, Cyclopropyl, Cyclopentyl oder Cyclohexyl bedeuten, oder geradkettiges oder verzweigtes Alkyl mit bis zu 14 Kohlenstoffatomen bedeuten, das gegebenenfalls durch Pyridyl substituiert ist, |
| $R^7$ | geradkettiges oder verzweigtes Alkyl mit bis zu 4 Kohlenstoffatomen bedeutet, |

A, B und D unabhängig voneinander
für eine direkte Bindung,
Prolin oder
für einen Rest der Formel

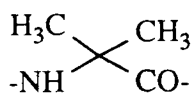

stehen, oder
für eine Gruppe der Formel

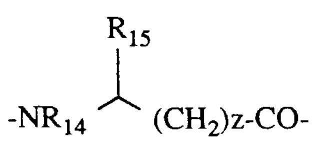

stehen,
worin

| | |
|---|---|
| z | die Zahl 0 oder 1 bedeutet, |
| $R^{14}$ | Wasserstoff oder Methyl bedeutet, |
| $R^{15}$ | Cyclopentyl, Cyclohexyl oder Wasserstoff bedeutet, oder geradkettiges oder verzweigtes Alkyl mit bis zu 4 Kohlenstoffatomen bedeutet, wobei das Alkyl gegebenenfalls durch Cyano, Methylthio, Hydroxy, Mercapto, Guanidyl, Amino, Carboxy oder $H_2N$-CO- substituiert sein kann, oder das Alkyl durch Cyclohexyl, Naphthyl oder Phenyl substituiert ist, das seinerseits durch Fluor, Chlor oder Alkoxy mit bis zu 4 Kohlenstoffatomen substituiert sein kann, oder das Alkyl durch Indolyl, Imidazolyl, Triazolyl, Pyridyl oder Pyrazolyl substituiert ist, wobei die NH-Funktion gegebenenfalls durch Methyl, Benzyloxymethylen oder tert.Butyloxycarbonyl (BOC) geschützt ist, |
| $R^1$ | für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann, |

E für einen Rest der Formel

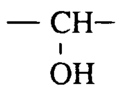 oder 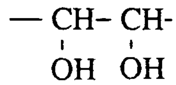

steht,

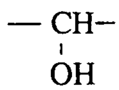 oder 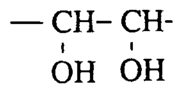

steht,

$R^2$ und $R^3$ gleich oder verschieden sind und für Wasserstoff oder für geradkettiges oder verzweigtes Alkyl mit bis zu 4 Kohlenstoffatomen stehen, oder

$R^2$ und $R^3$ gemeinsam mit dem Stickstoffatom und unter Einbezug der

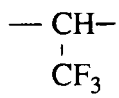

-Gruppe für einen Rest der Formel

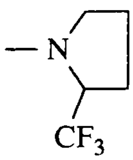 , 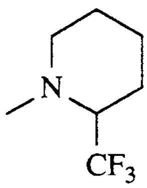 , 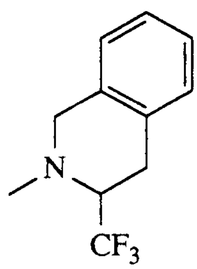 , 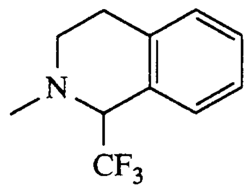 ,

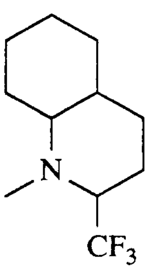 , 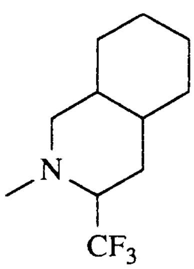 , 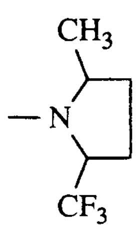 oder 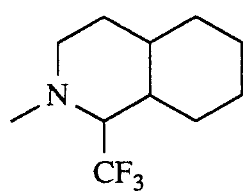

und deren physiologsich unbedenklichen Salze.

Außerdem wurden Verfahren zur Herstellung der erfindungsgemäßen Verbindungen der allgemeinen Formel (I) gefunden, dadurch gekennzeichnet, daß man im Fall, daß E für die

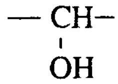

Gruppe steht,

[A] entweder Verbindungen der allgemeinen Formel (III)

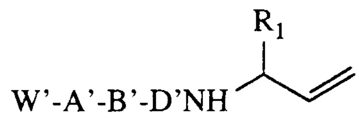

(III)

in welcher

| | |
|---|---|
| $R^1$ | die oben angegebene Bedeutung hat, |
| A', B' und D' | die oben angegebene Bedeutung von A, B und D haben aber nicht gleichzeitig für eine Bindung stehen und |
| W' | die oben angegebene Bedeutung von W hat, aber nicht für Wasserstoff steht, zunächst mit einer Epoxidierungsreaktion, gegebenenfalls mit Hilfe einer Base oder einer Phasentransferkatalyse in die Verbindungen der allgemeinen Formel (IV) |

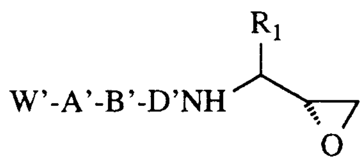

(IV)

in welcher

W', A', B', D' und $R^1$ die oben angegebene Bedeutung haben,

überführt und anschließend mit Verbindungen der allgemeinen Formel (V)

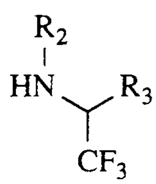

(V)

in welcher

$R^2$ und $R^3$ die oben angegebene Bedeutung haben,

in inerten Lösemitteln umsetzt und gegebenenfalls die Schutzgruppe W' abspaltet (W = H) oder austauscht,

oder

[B] direkt Verbindungen der allgemeinen Formel (IVa)

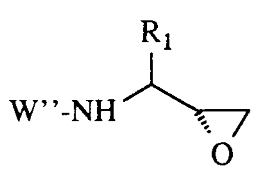

(IVa)

in welcher

$R^1$ die oben angegebene Bedeutung hat

und

W'' für eine Aminoschutzgruppe, vorzugsweise für Boc oder Z steht,

zunächst durch Umsetzung mit Verbindungen der allgemeinen Formel (V), in die Verbindungen der allgemeinen Formel (VI)

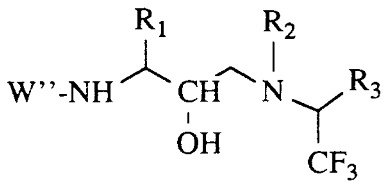

(VI)

in welcher
W'',$R^1$,$R^2$ und $R^3$ die oben angegebene Bedeutung haben,
überführt und anschließend entweder mit Verbindungen der allgemeinen Formel (VII), (VIIa), (VIII) oder (VIIIa)

W'-A-B-D-OH (VII); W'-A'-B'-D'-OH (VIIa);

W-X (VIII); oder $(G)_2O$ (VIIIa)

in welcher
W, W', A, A', B, B', D und D' die oben angegebene Bedeutung haben
X in Abhängigkeit von der Bedeutung des Substituenten W für Hydroxy oder Halogen, vorzugsweise für Chlor steht,
und
G für die $CF_3CO$- oder $H_3C$-CO-Gruppe steht,
im Fall der Verbindungen der allgemeinen Formel (VII) oder (VIIa) nach den in der Peptidchemie üblichen Bedingungen unter Aktivierung der Carbonsäure, gegebenenfalls in Anwesenheit einer Base und eines Hilfsstoffes, und jeweiliger Abspaltung der Schutzgruppen, in einem Schritt (VIIa) oder sukzessive (VII) in inerten Lösemitteln kondensiert, und gegebenenfalls den Substituenten W nach üblichen Methoden variiert,
und im Fall, daß E für die

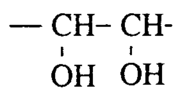

Gruppe steht,
[C] zunächst Verbindungen der allgemeinen Formel (IX)

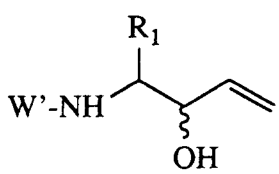

(IX)

in welcher
W' und $R^1$ die oben angegebene Bedeutung haben,
mit der unter [A] beschriebenen Epoxidierungsreaktion in die Verbindungen der allgemeinen Formel (X)

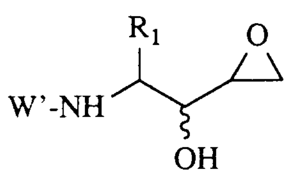

(X)

in welcher
W' und $R^1$ die oben angegebene Bedeutung haben,

überführt, diese mit den Verbindungen der allgemeinen Formel (V) unter Ringöffnung umsetzt und anschließend wie unter [B] beschrieben mit den Verbindungen der allgemeinen Formel (VII), (VIIa), (VIII) und/oder (VIIIa) umsetzt,
und gegebenenfalls eine Trennung der Diastereomere durchführt. Die erfindungsgemäßen Verfahren können durch folgende Formelschema beispielhaft erläutert werden:

[A]

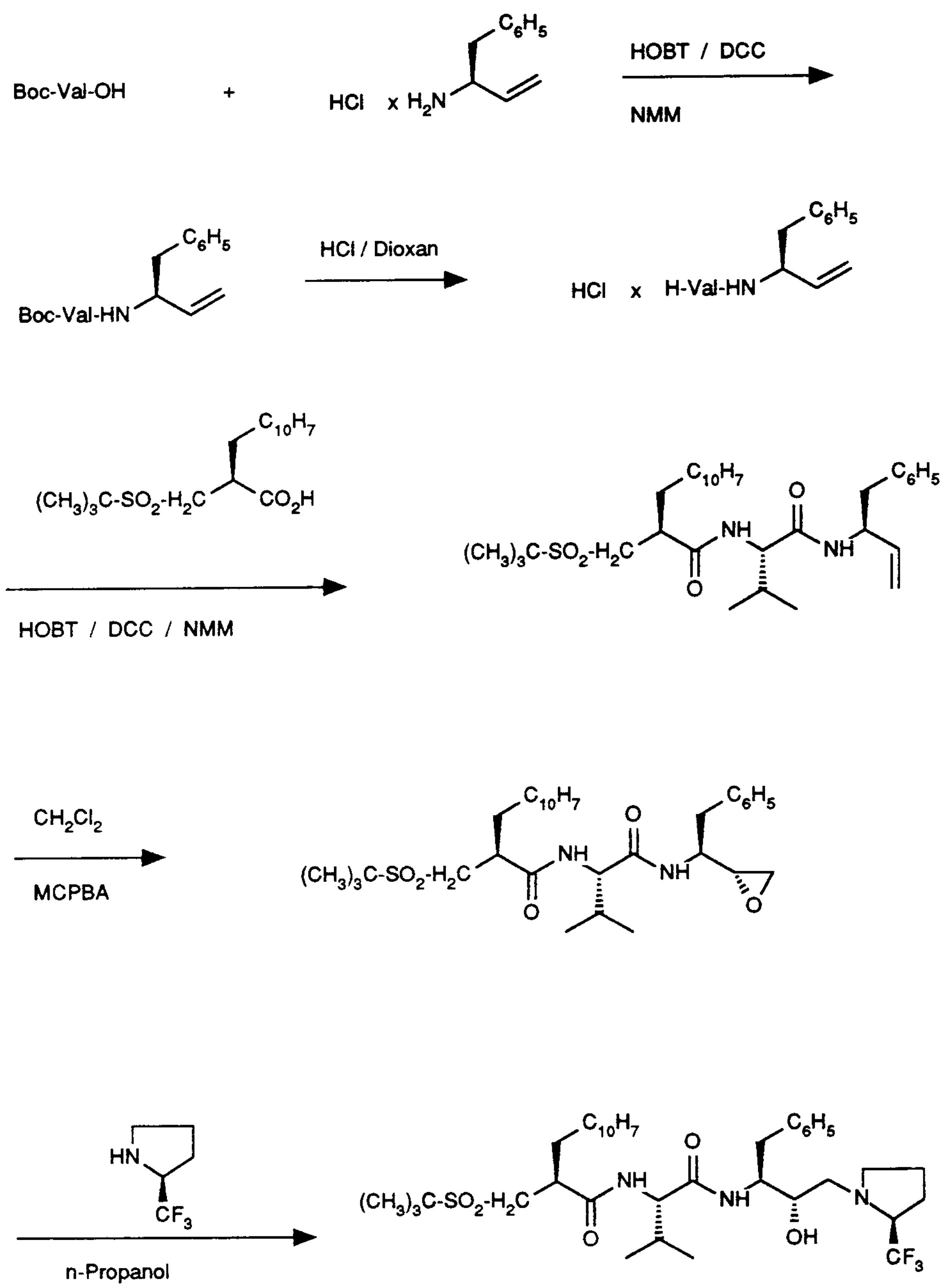

[B]

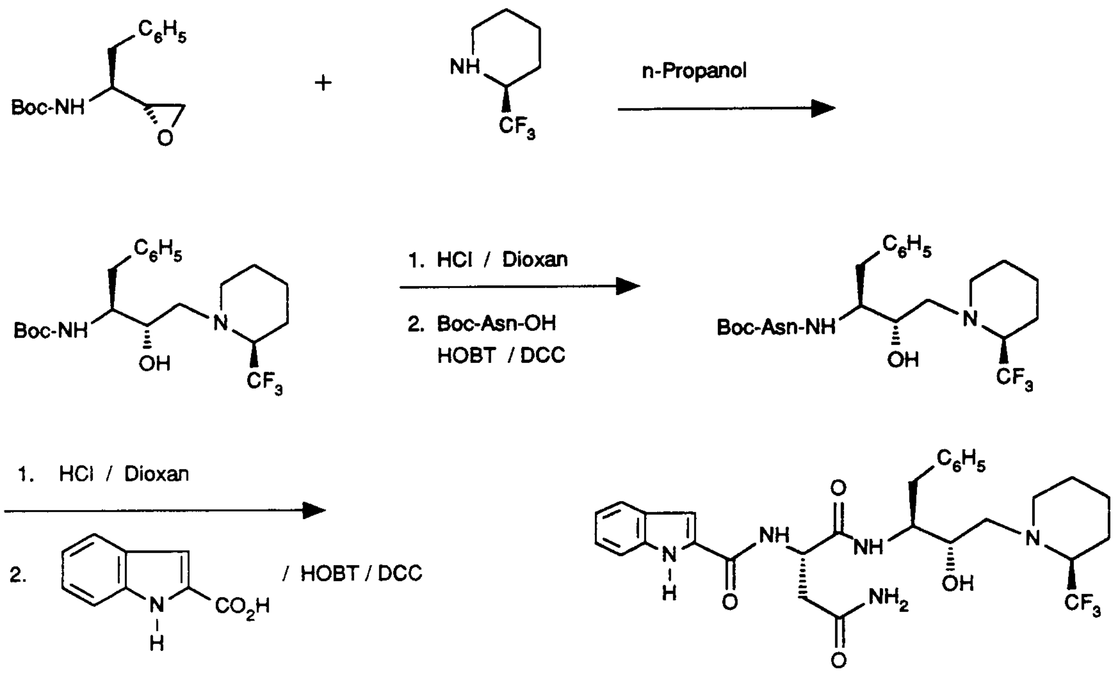


[B]

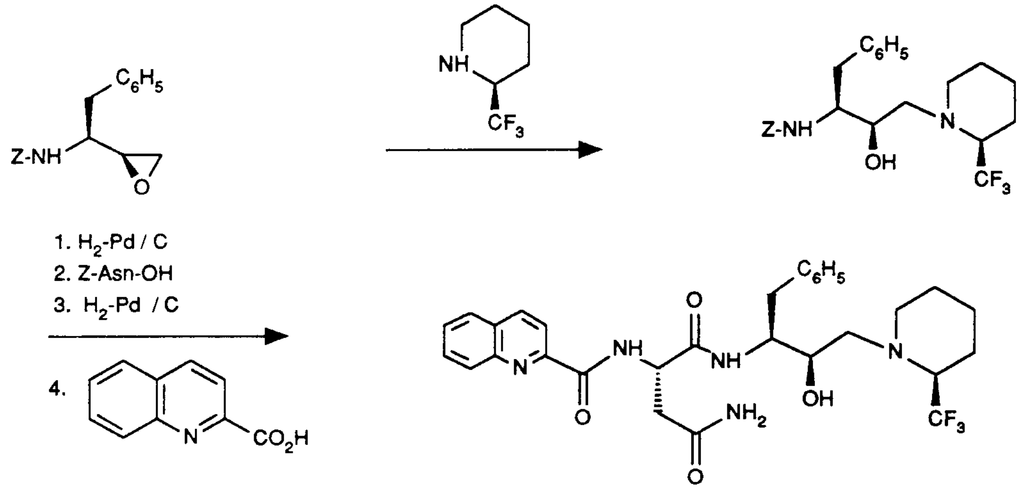


Z = Benzyloxycarbonyl

[C]

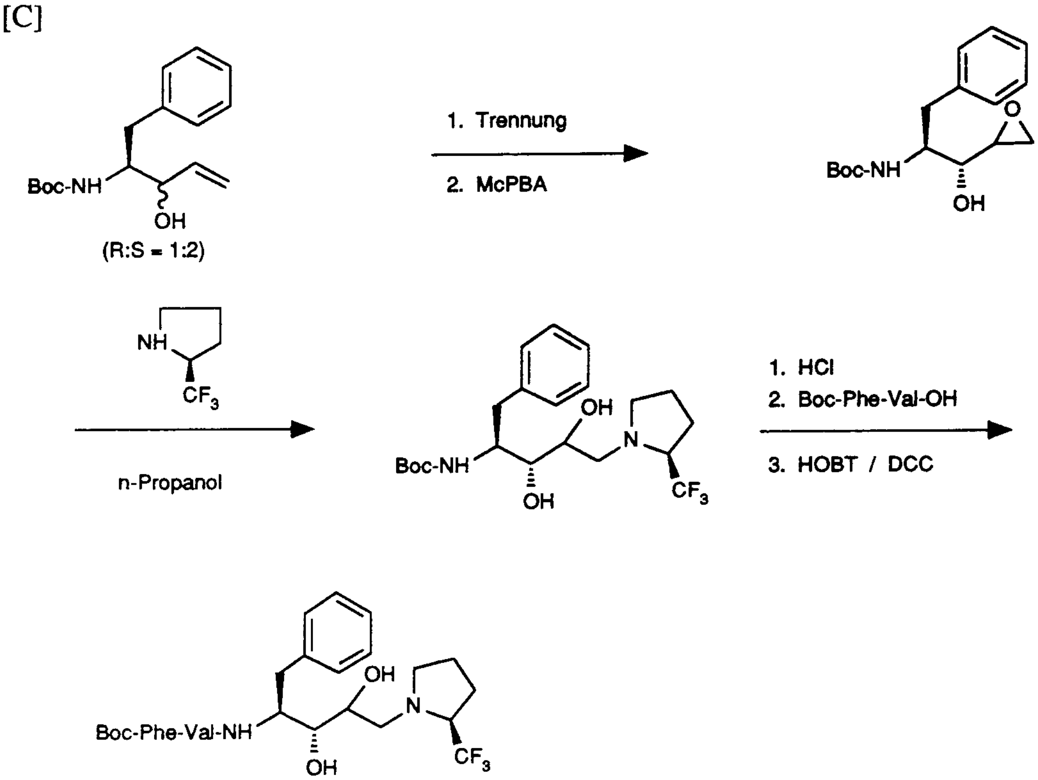


Als Lösemittel eignen sich die üblichen organischen Lösemittel, die sich unter den Reaktionsbedingungen nicht verändern. Hierzu gehören bevorzugt organische Lösemittel wie Alkohole, z.B. Methanol, Ethanol oder n-Propanol, Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Dichlorethan (DCE), Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dichlorethan, Dimethylformamid oder n-Propanol.

Als Reagentien für die Epoxidierung eignen sich die literaturbekannten Verbindungen wie beispielsweise m-Chlorbenzoesäure, Magnesiummonoperoxyphthalat, Dimethyldioxiran oder Methyl-(trifluormethyl)dioxiran. Bevorzugt sind m-Chlorperbenzoesäure und Magnesiummonoperoxyphthalat [vgl. P. Brongham et al., Synthesis (1987), 1015; W. Adam et al., J. Org. Chem. 52, 2800 (1987) und R. Curci et al., J. Org. Chem. 53, 3890 (1988)].

Wird die Epoxidierung mit Hilfe einer Phasentransfer-Katalyse durchgeführt so werden als Hilfsstoffe beispielsweise organische Ammoniumchloride oder -bromide wie beispielsweise Benzyltriethylammoniumchlorid oder -bromid, Methyltrioctylammoniumchlorid, Tetrabutylammoniumbromid, Tricaprylmethylammoniumchlorid (Aliquat 336) eingesetzt. Bevorzugt sind Benzyltriethylammoniumchlorid und -bromid.

Die Epoxidierung wird in einem Temperaturbereich von -10 °C bis +90 °C, bevorzugt von 0 °C bis +60 °C durchgeführt.

Die Reaktionen können sowohl bei Normaldruck als auch bei erhöhtem oder erniedrigtem Druck (beispielsweise 0,5 bis 5 bar), bevorzugt bei Normaldruck durchgeführtwerden.

Die Verbindungen der allgemeinen Formel (III) und (IIIa) sind teilweise bekannt oder neu und können hergestellt werden, indem man

[D] Verbindungen der allgemeinen Formel (XI)

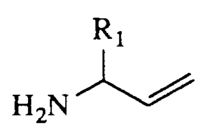

(XI)

in welcher
$R^1$ die oben angegebene Bedeutung hat,
mit Verbindungen der allgemeinen Formel (VIIa)

W'-A'-B'-D'-OH (VIIa)

in welcher
W', A', B' und D' die oben angegebene Bedeutung haben,
unter Aktivierung der Carbonsäure, gegebenenfalls in Anwesenheit einer Base und eines Hilfsstoffes, in einem Schritt oder sukzessive (je nach Bedeutung der Substituenten A', B' und D') umsetzt, oder
[E] Verbindungen der allgemeinen Formel (IIIa)

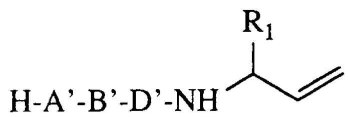

(IIIa)

in welcher
A', B', D' und $R^1$ die oben angegebene Bedeutung haben,
mit Verbindungen der allgemeinen Formeln (VIII) oder (VIIIa) nach den in der Peptidchemie üblichen Bedingungen in inerten Lösemitteln, gegebenenfalls in Anwesenheit einer Base unter Einführung der Schutzgruppe (W' oder W'') umsetzt.

Als Lösemittel eignen sich für alle Verfahrensschritte die üblichen inerten Lösemittel, die sich unter den Reaktionsbedindungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel zu verwenden. Besonders bevorzugt sind Dichlormethan, Dimethylformamid oder Tetrahydrofuran.

Einige wenige Verbindungen der allgemeinen Formeln (III) und (IIIa) sind literaturbekannt [vgl. J. Med. Chem. 34, 1225 (1991)].

Die Verbindungen der allgemeinen Formel (VII) und (VIIa) sind an sich bekannt und können durch Umsetzung eines entsprechenden Bruckstückes, bestehend aus einer oder mehreren Aminosäuregruppierungen, mit einer freien, gegebenenfalls in aktivierter Form vorliegenden Carboxylgruppe mit einem komplementierenden Bruchstück, bestehend aus einer oder mehreren Aminosäuregruppierungen, gegebenenfalls in aktivierter Form, und durch Wiederholung dieses Vorgangs mit entsprechenden Bruchstücken hergestellt werden; anschließend können gegebenenfalls Schutzgruppen abgespalten oder gegen andere Schutzgruppen ausgetauscht werden [vgl. Houben-Weyl, Methoden der organischen Chemie, Synthese von Peptiden II, 4. Aufl. Bd. 15/1, 15/2, Georg Thieme Verlag, Stuttgart].

Als Hilfsstoffe für die jeweiligen Peptidkupplungen und für die Einführung des Restes W (VIII) und (VIIIa) werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N'-Diethyl-, N,N'-Diisopropyl-, N,N'-Dicyclohexylcarbodiimid, N-(3-Dimethylaminoisopropyl)-N'-ethyl-carbodiimid-Hydrochlorid, N-Cyclohexyl-N'(2-morpholinoethyl)-carbodiimid-metho-p-toluolsulfonat(CMCT), oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphat oder 1-Hydroxybenzotriazol.

Die Verbindungen der allgemeinen Formel (XI) sind an sich bekannt oder können nach literaturbekannten Methoden hergestellt werden [vgl. J.R. Luly et al., J. Org. Chem. 52, (1987), 1487].

Die Verbindungen der allgemeinen Formel (IV) sind bis auf wenige Ausnahmen [vgl. J. Med. Chem. 34, 1225, (1991)] neu und können dann nach dem oben aufgeführten Verfahren hergestellt werden.

Die Verbindungen der allgemeinen Formel (V) sind im Fall, daß $R^2$ und $R^3$ jeweils für einen oben definierten offenkettigen Rest stehen, teilweise bekannt [vgl. J. Org. Chem. 27, 1406 (1962)] oder können nach den dort publizierten Verfahren hergestellt werden. Im Fall, daß $R^2$ und $R^3$, ebenfalls wie oben

definiert, gemeinsam mit dem Stickstoffatom ein heterocyclisches oder benzokondensiertes heterocyclisches 5- oder 6-gliedriges, gegebenenfalls durch eine Methylgruppe substituiertes Ringsystem bilden, sind die Verbindungen der allgemeinen Formel (V) teilweise bekannt [vgl. Isv. Akad. Nauk. SSSR, 1422 (1987); J. Org. Chem. 27, 1406 (162); US 39 563 33; US 39 270 00; US 38 552 28] oder neu und können dann beispielsweise durch Umsetzung mit Schwefeltetrafluorid und den entsprechenden Aminosäuren in Lösemittel, wie vorzugsweise Fluorwasserstoffsäure hergestellt werden.

Die Verbindungen der allgemeinen Formel (IVa) sind teilweise bekannt ($R^1$ = $-CH_2-C_6H_5$; $-CH_2-C_6H_{11}$) [vgl. J.R. Luly et al., J. Org. Chem. 52, 1487 (1987)] oder können nach den dort angegebenen Methoden hergestellt werden.

Die Verbindungen der allgemeinen Formel (VI) sind ebenfalls neu und können nach dem unter [B] aufgeführten Verfahren hergestellt werden.

Die Verbindungen der allgemeinen Formeln (VIII) und (VIIIa) sind bekannt oder können nach literaturbekannten Methoden hergestellt werden [vgl. EP 402 646].

Die Verbindungen der allgemeinen Formel (IX) sind teilweise bekannt ($R^1$ = $-CH_2-C_6H_5$; $-CH_2-C_6H_{11}$) [vgl. J. Org. Chem. 50, 5399 (1985); EP 202 577; EP 337 714 oder J. Med. Chem. 32, 1371 (1989)] oder neu und können aber in diesem Fall in Analogie zu den oben aufgeführten publizierten Verfahren, beispielsweise durch Umsetzung des entsprechenden Aldehyds mit Vinylmagnesiumbromid in inerten Lösemitteln, vorzugsweise Tetrahydrofuran, hergestellt werden.

Ebenso sind die Verbindungen der allgemeinen Formel (X) teilweise bekannt [vgl. EP 230 266; EP 189 203; EP 311 012 und J. Med. Chem. 32, 1371 (1989)] oder neu (z.B. $R^1$ = $-CH_2-C_6H_5$) und können dann in Analogieverfahren dargestellt werden.

Es wurde überraschend gefunden, daß die Verbindungen der allgemeinen Formel (I) eine außerordentlich starke Wirkung gegen Retroviren besitzen. Dies wird mit einem HIV-spezifischen Protease-Enzymtest belegt.

Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen sich auf die Substanzkonzentration, die unter den oben aufgeführten Testbedingungen eine 50%ige Hemmung der Protease-Aktivität bewirkt.

Enzym assay, HIV-1/HIV-2

Tabelle I

| Bsp.-Nr. | $IC_{50}$ (RP-HPLC) (M) | |
|---|---|---|
| | HIV-1 | HIV-2 |
| 12 | $5 \times 10^{-9}$ | $10^{-9}$ |
| 14 | $10^{-8}$ | not tested |
| 18 | $5 \times 10^{-9}$ | not tested |
| 19 | $10^{-8}$ | not tested |
| 22 | $10^{-9}$ | not tested |
| 24 | $5 \times 10^{-8}$ | not tested |
| 31 | $10^{-10}$ | not tested |
| 35 | $10^{-9}$ | not tested |
| 36 | $5 \times 10^{-7}$ | not tested |
| 37 | $5 \times 10^{-9}$ | not tested |
| 43 | $5 \times 10^{-8}$ | not tested |
| 45 | $15^{-10}$ | not tested |
| 72 | $10^{-8}$ | not tested |
| 75 | $5 \times 10^{-10}$ | not tested |

Außerdem zeigten die erfindungsgemäßen Verbindungen Wirkung in Lentivirus infizierten Zellkulturen. Dies konnte am Beispiel des HIV-Virus gezeigt werden.

HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Die Virusadsorptionslösung wurde zentrifugiert und das infizierte Zellpellet in Wachstumsmedium aufgenommen, so daß 1 x $10^5$ Zellen pro ml eingestellt waren. Die derart infizierten Zellen wurden zu 1 x $10^4$ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 10-fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 Syncytien, währen die unbehandelte Zellkontrolle keine Syncytien aufwies.

Die $IC_{50}$-Werte wurden als die Konzentration der behandelten und infizierten Zellen ermittelt, bei der 50% (ca. 10 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

Tabelle II

| Bsp.-Nr. | $IC_{50}$ (µM) | |
|---|---|---|
| | [PBL] | [H-9] |
| 8 | 5 | - |
| 12 | 0,14 | 0,53 |
| 14 | 1,4 | - |
| 18 | | 1,1 |
| 20 | 0,5 - 1 | 2,0 |
| 24 | 2,5 | |
| 26 | - | 1,5 |
| 37 | - | 2,5 |
| 45 | 5 | - |
| 69 | - | 16 |
| 71 | - | 6,5 |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:
Infektionen mit

a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2,3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

<u>Anhang zum experimentellen Teil</u>

<u>I. Liste der verwendeten Laufmittelgemische zur Chromatographie:</u>

| | |
|---|---|
| I | Dichlormethan: Methanol |
| II | Toluol : Ethylacetat |
| III | Acetonitril : Wasser |
| IV | Dichlormethan: Methanol: Ammoniak (9:1:0,1) |
| V | Toluol: Acetonitril |

<u>II. Aminosäuren</u>

Im allgemeinen erfolgt die Bezeichnung der Konfiguration durch das Voraustellen eines L bzw. D vor der Aminosäureabkürzung, im Fall des Racemats durch ein D,L-, wobei zur Vereinfachung bei L-Aminosäuren die Konfigurationsbezeichnung unterbleiben kann und dann nur im Fall der D-Form bzw. des D,L-Gemisches eine explizierte Bezeichnung erfolgt.

| | |
|---|---|
| Ala | L-Alanin |
| Arg | L-Arginin |
| Asn | L-Asparagin |
| Asp | L-Asparaginsäure |
| Cys | L-Cystein |
| Gln | L-Glutamin |
| Glu | L-Glutaminsäure |
| Gly | L-Glycin |
| His | L-Histidin |
| Ile | L-Isoleucin |

| | |
|---|---|
| Leu | L-Leucin |
| Lys | L-Lysin |
| Met | L-Methionin |
| Pro | L-Prolin |
| Phe | L-Phenylalanin |
| Ser | L-Serin |
| Thr | L-Threonin |
| Trp | L-Tryptophan |
| Tyr | L-Tyrosin |
| Val | L-Valin |

III. Abkürzungen

| | |
|---|---|
| Z | Benzyloxycarbonyl |
| Boc | tert.Butyloxycarbonyl |
| CMCT | 1-Cyclohexyl-3-(2-morpholino-ethyl)-carbodiimidmetho-p-toluolsulfonat |
| DCC | Dicyclohexylcarbodiimid |
| DMF | Dimethylformamid |
| HOBT | 1-Hydroxybenzotriazol |
| Mir | Miristoyl |
| Ph | Phenyl |
| THF | Tetrahydrofuran |
| Cha | Cyclohexylalanin |
| MCPBA | m-Chlorperbenzoesäure |
| MMPP | Magnesiummonoperoxyphthalat Hexahydrat |
| Aib | 2-Amino-2-methylpropionsäure |

Ausgangsverbindungen

Beispiel I

(S)-2-Amino-1-phenylbut-3-en Hydrochlorid

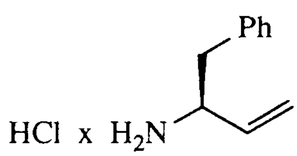

Eine Lösung von 5,00 g (20,21 mmol) (S)-2-(tert.Butoxycarbonylamino-1-phenylbut-3-en [J.R. Luly et al., J. Org. Chem. 52, 1487 (1987)] in 100 ml einer 4 N Lösung gasförmigen Chlorwasserstoffs in wasserfreiem Dioxan wurde 30 min bei Raumtemperatur gerührt. Danach gab man 15 ml Toluol zu und engte im Vakuum ein. Dieser Vorgang wurde noch zweimal wiederholt, dann wurde der Rückstand mit wenig Ether verrieben, abgesaugt und im Hochvakuum über KOH getrocknet. Man erhielt 3,69 g (99% der Theorie) der Titelverbindung als farblose Kristalle.

$R_f$ = 0,67, Laufmittelgemisch IV

MS (DCI, $NH_3$): m/z = 148 ($M+H^+$)

Beispiel II

(S)-2-Amino-1-cyclohexylbut-3-en Hydrochlorid

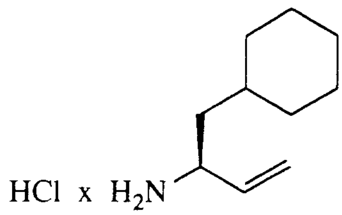

Wie für Beispiel I beschrieben erhielt man aus 5.07 g (20,00 mmol) (S)-2-(tert.Butoxycarbonylamino-1-cyclohexylbut-3-en [J.R. Luly et al., J. Org. Chem. 52, 1487 (1987)] 3,76 g (99% der Theorie) der Titelverbindung als farblose Kristalle.
Schmp.: 232-233 °C
$R_f$ = 0,42, III (9:1)
MS (EI, 70 eV) m/z = 153 $(M)^+$

Beispiel III

(2S)-2-[N-(tert.Butoxycarbonyl-L-valinyl)]amino-1-phenylbut-3-en

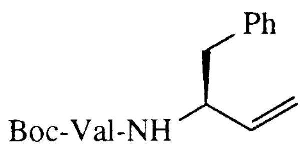

Eine auf 0 °C gekühlte, gerührte Lösung von 4,81 g (22,13 mmol) N-(tert.Butoxycarbonyl)-L-valin und 3,29 g (24,35 mmol) HOBT in 40 ml wasserfreiem Dichlormethan wurde mit 5,29 g (25,65 mmol) DCC versetzt und 5 min gerührt. Danach wurde eine Lösung von 3,70 g (20.12 mmol) der Verbindung aus Beispiel I und 8,85 ml (80,48 mmol) N-Methylmorpholin in 30 ml Dichlormethan zugetropft. Das Kühlbad wurde entfernt und die Reaktionsmischung durfte 2 h bei Raumtemperatur rühren. Das Ende der Reaktion wurde dünnschichtchromatographisch festgestellt. Man trennte den entstandenen Harnstoff durch Filtration ab, engte das Filtrat im Vakuum ein und reinigte das Rohprodukt durch Chromatographie an 450 g Kieselgel (Dichlormethan : Methanol 95:5). Man erhielt 6,07 g (87% der Theorie) der Titelverbindung als farblosen Schaum.
$R_f$ = 0,41, IV
MS (DCI, $NH_3$): m/z = 347 $(M+H)^+$.

Beispiel IV

(2S)-2-[(N-(tert.Butoxycarbonyl)-L-valinyl)]amino-1-cyclohexylbut-3-en

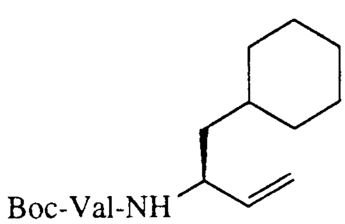

Wie für Beispiel III beschrieben erhielt man aus 3,60 g (19,00 mmol) der Verbindung aus Beispiel II und 4,63 g (21,3 mmol) Boc-Val-OH 4,33 g (65% der Theorie) der Titelverbindung als farblose Kristalle.
Schmp.: 127-128 °C (Zers.)

$R_f$ = 0,27, II (9:1)
MS (DCI, $NH_3$) m/z = 353 $(M+H)^+$

Beispiel V

(2S)-1-Phenyl-2-(N-L-valinyl)aminobut-3-en Hydrochlorid

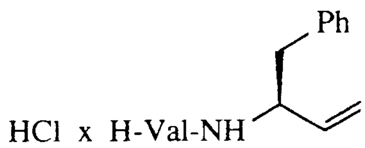

Wie für Beispiel 1 beschrieben erhielt man aus 6,08 g (17,53 mmol) der Verbindung aus Beispiel III 4,90 g (99% der Theorie) der Titelverbindung als farbloses Pulver.
$R_f$ = 0,36, I (9:1)

Beispiel VI

(2S)-1-Cyclohexyl-2-(N-L-valinyl)aminobut-3-en Hydrochlorid

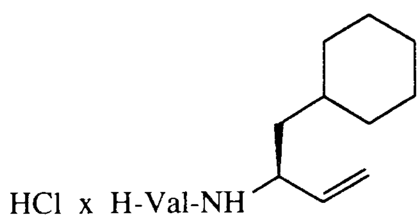

Wie für Beispiel I beschrieben erhielt man aus 4,32 g (12,30 mmol) der Verbindung aus Beispiel IV 3,37 g (95% der Theorie) der Titelverbindung als farbloses Pulver.
Schmp.: 169-170 °C
$R_f$ = 0,48, III (9:1)
MS (DCI, $NH_3$) m/z = 253 $(M+H)^+$

Beispiel VII

(2S)-2-[N-(2S)-3-(tert.Butylsulfonyl)-2-(1-naphthylmethyl)propanoyl]-L-valinyl]amino-1-phenylbut-3-en

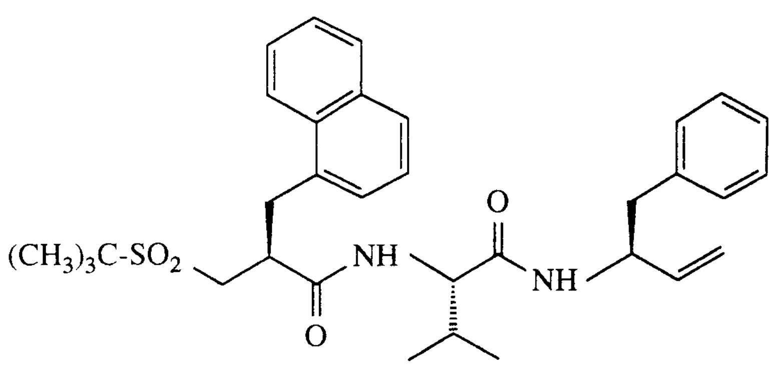

Eine auf 0 °C gekühlte, gerührte Lösung von 1,50 g (4,47 mmol) (2S)-3-tert.Butylsulfonyl-2-(1-naphthylmethyl)-propionsäure [hergestellt nach H. Bühlmayer et al., J. Med. Chem. 31, 1839 (1988)] und 0,66g (4,92 mmol) HOBT in 15 ml wasserfreiem Dichlormethan wurde mit 0,97 g (4,69 mmol) DCC versetzt und 5 min gerührt. Danach wurde eine Lösung von 1,15 g (4,07 mmol) der Verbindung aus Beispiel V und 1,80 ml

(16,27 mmol) N-Methylmorpholin in 10 ml Dichlormethan zugetropft und die Reaktion durfte 1 h bei Raumtemperatur rühren. Man trennte den entstandenen Harnstoff durch Filtration ab, engte das Filtrat im Vakuum ein und reinigte das Rohprodukt durch Chromatographie an 270 g Kieselgel (Dichlormethan : Methanol 95:5). Man erhielt 2.01 g (88% der Theorie) der Titelverbindung als farblosen Schaum.

$R_f$ = 0,47, I (95:5)

MS (FAB) m/z = 563 $(M+H)^+$

Wie für Beispiel VII beschrieben, erhielt man durch Kupplung der entsprechenden Säuren mit den Aminhydrochloriden (Startmaterialien) die folgenden Produkte (Tabelle 1):

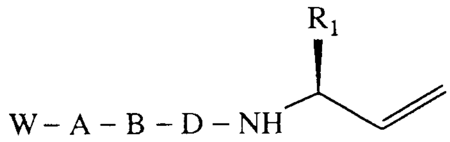

Tabelle 1

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| VIII | 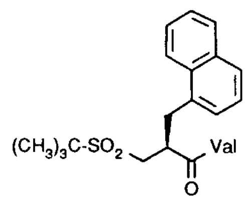 | $C_6H_{11}-CH_2$ | 76 | 569 | 0,57, I (1:1) | VI |
| IX | 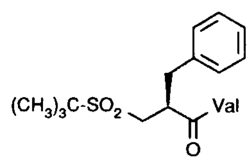 | $C_6H_5-CH_2$ | 92 | 513 | 0,48, I (95:5) | V |
| X | 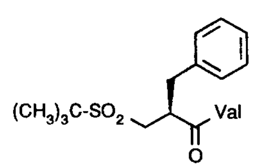 | $C_6H_{11}-CH_2$ | 75 | 519 | 0,56, II (1:1) | VI |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XI | $CH_3$-$SO_2$-Phe-Val | $CH_2$-$C_6H_5$ | 62 | 472 | 0,12, 1 (95:5) | V |
| XII | $CH_3$-$SO_2$-Phe-Val | $CH_2$-$C_6H_{11}$ | 58 | 478 | 0,50, II (1:1) | VI |
| XIII | 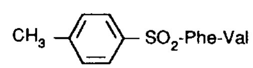 | $CH_2$-$C_6H_5$ | 29 | 548 | 0,53, 1 (95.5) | V |
| XIV | 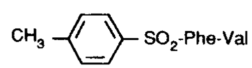 | $CH_2$-$C_6H_{11}$ | 21 | 554 | 0,70, II (1:1) | VI |
| XV | $(CH_3)_3C$-$CH_2$-CO-Phe-Val | $CH_2$-$C_6H_5$ | 80 | 492 | 0,31, 1 (95:5) | V |
| XVI | $(CH_3)_3C$-$CH_2$-CO-Phe-Val | $CH_2$-$C_6H_{11}$ | 57 | 498 | 0,25, I (95:5) | VI |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XVII | Boc-Phe-Val | $CH_2$-$C_6H_5$ | 74 | 494 | 0,44, I (95:5) | V |
| XVIII | Boc-Phe-Val | $CH_2$-$C_6H_{11}$ | 62 | 500 | 0,38, I (95:5) | VI |
| XIX | Z-Phe-Val | $CH_2$-$C_6H_5$ | 65 | 528 | 0,56, I (95:5) | V |
| XX | Z-Phe-Val | $CH_2$-$C_6H_{11}$ | 75 | 534 | 0,25, I (95:5) | VI |
| XXI | 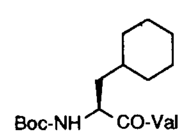 | $CH_2$-$C_6H_5$ | 84 | 500 | 0,43, I (95:5) | V |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXII | 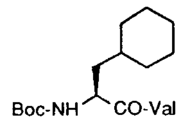 | $CH_2$-$C_6H_{11}$ | 70 | 506 | 0,38, I (95:5) | VI |
| XXIII | $CH_3(CH_2)_{12}$-CO-Phe-Val | $CH_2$-$C_6H_5$ | 82 | 604 | 0,34, I (95:5) | V |
| XXIV | 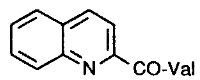 | $CH_2$-$C_6H_5$ | 54 | 402 | 0,61, I (95:5) | V |
| XXV | 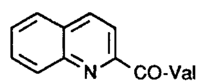 | $CH_2$-$C_6H_{11}$ | 54 | 408 | 0,21, II (4:1) | VI |
| XXVI | 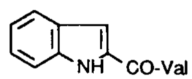 | $CH_2C_6H_5$ | 96 | 390 | 0,51, I (95:5) | V |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXVII | Boc-Aib | $CH_2$-$C_6H_5$ | 52 | 333 | 0,51, I (97:3) | I |
| XXVIII | Boc-Phe-Gly-Gly | $CH_2$-$C_6H_5$ | 77 | 509 | 0,45, IV | I |
| XXIX | Boc-Ser-Phe | $CH_2$-$C_6H_5$ | 34 | 482 | 0,45, I (9:1) | I |
| XXX | Boc-Asn | $CH_2$-$C_6H_5$ | 38 | 362 | 0,13, I (95:5) | I |
| XXXI | Boc-Ile | $CH_2$-$C_6H_5$ | 64 | 361 | 0,58, I (97:3) | I |
| XXXII | 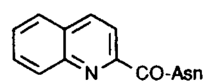 | $CH_2$-$C_6H_5$ | 39 | 417 | 0,26, I (95:5) | XXXVII |
| XXXIII | 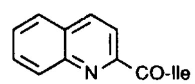 | $CH_2$-$C_6H_5$ | 90 | 416 | 0,28, II (4:1) | XXXVIII |

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XXXIV | 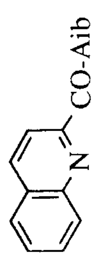 | $CH_2$-$C_6H_5$ | 70 | 388 | 0,48, II (7:3) | XXXIX |
| XXXV | 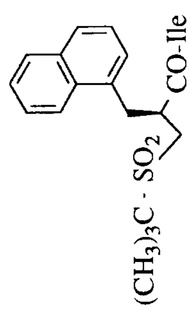 | $CH_2$-$C_6H_5$ | 72 | 576 | 0,18, II (7:3) | XXXVIII |
| XXXVI | 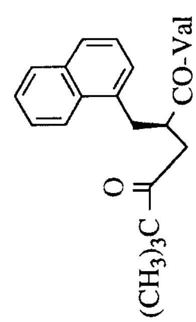 | $CH_2$-$C_6H_5$ | 78 | 527 | 0,39, II (7:3) | V |

Wie für Beispiel I beschrieben erhielt man durch Abspaltung der tert.Butoxycarbonyl-Schutzgruppe aus den entsprechenden Startmaterialien die folgenden Produkte (Tabelle 2):

Tabelle 2

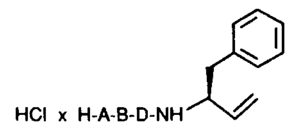

| Beispiel-Nr. | H-A-B-D- | Ausbeute (%) | MS(FAB) m/z $(M+H)^+$ | $R_f$/Laufmittel Verhältnis | Startmaterial aus Beispiel |
|---|---|---|---|---|---|
| XXXVII | H-Asn | 92 | 262 | 0,06, I (9:1) | XXX |
| XXXVIII | H-Ile | 91 | 261 | 0,58, I (95:5) | XXXI |
| XXXIX | H-Aib | 88 | 233 | 0,35, I (97:3) | XXVII |

Beispiel XL

2-{1-[N[(tert.Butylacetyl)-L-phenylalanyl]-L-valinyl]amino-2-phenyl-(1S)-ethyl}oxiran

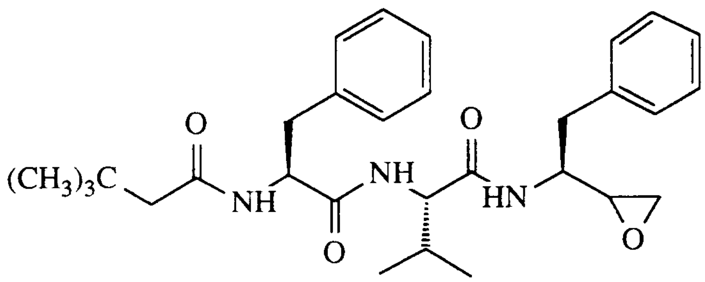

Eine gerührte, auf 0 ° C gekühlte Suspension von 250 mg (0,60 mmol) der Verbindung aus Beispiel XV in 3 ml Dichlormethan wurde portionsweise mit 259 mg (1,20 mmol - 2 equiv.) m-Chlorperbenzoesäure (80%ig) (MCPBA) versetzt und 2 h bei dieser Temperatur gerührt. Danach wurden weitere 130 mg (0,60 mmol - 1 equiv.) MCPBA zugegeben und noch 1 h bei Raumtemperatur nachgerührt. Danach wurden 10 ml Ethylacetat zugegeben und die Reaktionsmischung wurde in 20 ml einer 10%igen $Na_2SO_3$-Lösung eingerührt. Die organische Phase wurde abgetrennt, 3 mal mit 10 ml $NaHCO_3$-Lösung gewaschen und über $MgSO_4$ getrocknet. Nach Abdampfen des Lösemittels im Vakuum und Verreiben des Rückstandes mit wenig Ether/Pentan erhielt man die 253 mg (83% der Theorie) der Titelverbindung als farbloses Pulver.
Schmp.: 168 ° C (Zers.)
$R_f$ = 0,26, I (97:3)
MS (FAB) m/z = 508 $(M+H)^+$

Beispiel XLI

(2S)-{1-[N-[(2S)-Benzyl-3-(tert.Butylsulfonyl)propanoyl]-L-valinyl]amino-2-phenyl-(1S)-ethyl}oxiran

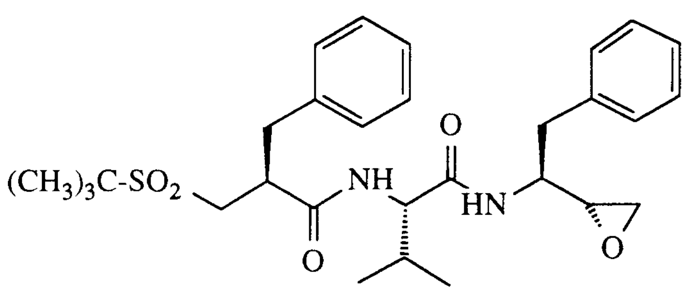

Eine Suspension von 345 mg (0,67 mmol) der Verbindung aus Beispiel IX, 8 mg (5 mol-%) Benzyltriethylammoniumchlorid und 668 mg (1,35 mmol) Magnesiummonoperoxyphthalat Hexahydrat (MMPP) in 3 ml Chloroform wurde durch Zugabe von 1N NaOH-Lösung auf pH 5 gestellt und 16 h zum Rückfluß erhitzt, wobei durch Zugabe kleiner Mengen von 1N NaOH etwa pH 5 gehalten wurde. Nach dem Abkühlen wurde die Reaktionsmischung abgesaugt und das Filtrat mit 10 ml Wasser, 10 ml 10%ige $Na_2SO_3$-Lösung und 10 ml verdünnte $NaHCO_3$-Lösung gewaschen und über Magnesiumsulfat getrocknet. Nach Abdampfen des Lösemittels im Vakuum und Chromatographie des Rückstands an 15 g Kieselgel (Toluol : Ethylacetat 1:1) erhielt man 131 mg (37% der Theorie) der Titelverbindung als farblosen Hartschaum.
$R_f$ = 0,21, II (1:1)
MS (FAB) m/z = 529 $(M+H)^+$
HPLC: Gemisch der diastereomeren Epoxide (1S): (1R) = 16:1
Wie für Beispiel XL beschrieben, erhielt man die folgenden Epoxide (Tabelle 3):

Tabelle 3:

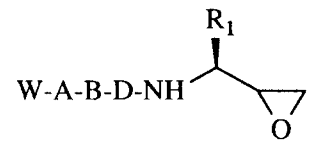

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XLII | Z-Phe-Val | $CH_2$-$C_6H_5$ | 81 | 544 | 0,39 , I (95:5) | XIX |
| XLIII | Boc-Cha-Val | $CH_2$-$C_6H_5$ | 47 | 516 | 0,53, I (95:5) | XXI |
| XLIV | 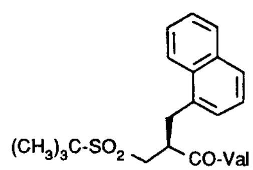 | $CH_2$-$C_6H_5$ | 4 | 579 | 0,16, II (4:6) | VII |
| XLV | 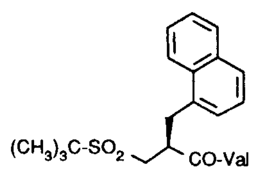 | $CH_2$-$C_6H_5$ | 38 | 579 | 0,12, II (4:6) | VII |

Fortsetzung Tabelle 3:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| XLVI | 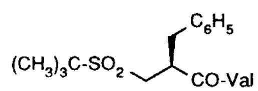 | $CH_2$-$C_6H_5$ | 43 | 529 | 0,21, II (1:1) | IX |
| XLVII | $CH_3$-$SO_2$-Phe-Val | $CH_2$-$C_6H_5$ | 10 | 488 | 0,22, II (1:1) | XI |
| XLVIII | $CH_3$-$SO_2$-Phe-Val | $CH_2$-$C_6H_5$ | 39 | 488 | 0,15, II (1:1) | |
| XLIX | Boc-Phe-Val | $CH_2$-$C_6H_5$ | 46 | 510 | 0,05, I (97:3) | XVII |

Fortsetzung Tabelle 3:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| L | 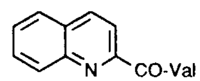 | $CH_2$-$C_6H_5$ | 66 | 418 | 0,25, II (7:3) | XXIV |
| LI | 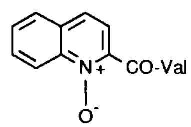 | $CH_2$-$C_6H_5$ | 22 | 418 | 0,12, II (7:3) | |
| LII | $CH_3(CH_2)_{12}$-CO-Phe-Val | $CH_2$-$C_6H_5$ | 55 | 620 | 0,33, I (97:3) | XXIII |
| LIII | 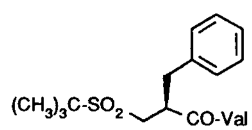 | $CH_2$-$C_6H_{11}$ | 54 | 535 | 0,12, I (95:5) | X |
| LIV | 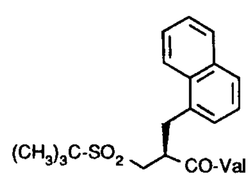 | $CH_2$-$C_6H_{11}$ | 53 | 585 | 0,28, I (97:3) | VIII |

Fortsetzung Tabelle 3:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| LV | Boc-Phe-Val | $CH_2$-$C_6H_{11}$ | 51 | 516 | 0,11, I (97:3) | XVIII |
| LVI | $CH_3$-$SO_2$-Phe-Val | $CH_2$-$C_6H_{11}$ | 55 | 494 | 0,13, I (97:3) | XII |
| LVII | $(CH_3)_3C$-$CH_2$-CO-Phe-Val | $CH_2$-$C_6H_{11}$ | 48 | 514 | 0,12, I (97:3) | XVI |
| LVIII | Boc-Cha-Val | $CH_2$-$C_6H_{11}$ | 55 | 522 | 0,19, I (97:3) | XXII |
| LIX | Z-Phe-Val | $CH_2$-$C_6H_{11}$ | 61 | 550 | 0,34, II (6:4) | XX |
| LX | Z-Phe-Val | $CH_2$-$C_6H_{11}$ | 8 | 550 | 0,14, II (6:4) | XX |

Fortsetzung Tabelle 3:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| LXI | Boc-Ser-Phe | $CH_2$-$C_6H_5$ | 38 | 500 | 0,12, I (9:1) | XXIX |
| LXII | Boc-Phe-Gly-Gly | $CH_2$-$C_6H_5$ | 49 | 525 | 0,41, I (9:1) | XXVIII |
| LXIII | 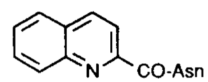 | $CH_2$-$C_6H_5$ | 17 | 433 | 0,11, I (95:5) | XXXII |
| LXIV | 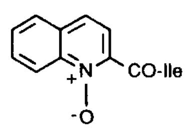 | $CH_2$-$C_6H_5$ | 33 | 448 | 0,16, II (3:2) | XXXIII |
| LXV | 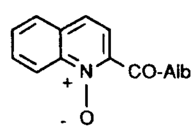 | $CH_2$-$C_6H_5$ | 68 | 420 | 0,13, II (3:2) | XXXIV |

Fortsetzung Tabelle 3:

| Beispiel-Nr. | W-A-B-D- | $R^1$ | Ausbeute (%) | MS(FAB) m/z $(M+H)^+$ | $R_f$/Laufmittel Verhältnis | Startmaterial aus Beispiel |
|---|---|---|---|---|---|---|
| LXVI | 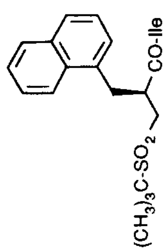 | $CH_2-C_6H_5$ | 89 | 593 | 0,15, II (3:2) | XXXV |
| LXVII | 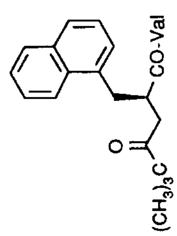 | $CH_2-C_6H_5$ | 21 | 543 | 0,26, II (7:3) | XXXVI |
| LXVIII | 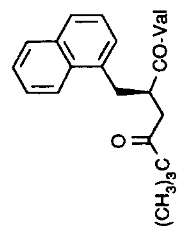 | $CH_2-C_6H_5$ | 22 | 543 | 0,16, II (7:3) | XXXVII |

Beispiel LXIX

2-{(1R,2S)-3-[N-(tert.Butoxycarbonyl)amino]-1-hydroxy-3-phenylpropyl]oxiran

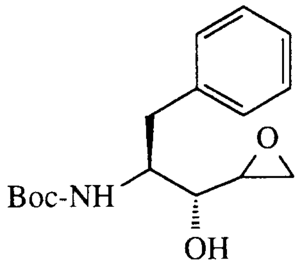

Eine gerührte, auf 0 °C gekühlte Lösung von 2,77 g (18,0 mmol) (3S, 4S)-4-[N-(tert.Butoxycarbonyl)amino]-3-hydroxy-5-phenylpenten [vgl. G.J. Hansen et al., J. Org. Chem. 50, 5399 (1985)] in 28 ml Dichlormethan wurde portionsweise mit 5,18 g (30 mmol - 3 equiv.) m-Chlorperbenzoesäure (80%ig) (MCPBA) versetzt und 2 h bei 0 °C und 1 h bei Raumtemperatur gerührt. Danach wurden 50 ml Ethylacetat zugegeben und die Reaktionsmischung wurde in 100 ml einer 20%igen $Na_2SO_3$-Lösung eingerührt. Die organische Phase wurde abgetrennt, 3 mal mit 50 ml 20%iger $Na_2CO_3$-Lösung gewaschen und über $MgSO_4$ getrocknet. Nach Abdampfen des Lösemittels im Vakuum und Chromatographie des Rückstandes an 110 g Kieselgel (Toluol : Ethylacetat 4:1) erhielt man 1,87 g (64% der Theorie) der Titelverbindung als farblose Kristalle.
Schmp.: 102 °C
$R_f$ = 0,21, II (7:3)
MS (FAB): m/z = 294 $(M+H)^+$

Beispiel LXX

2-{(1S,2S)-2-[N(tert.Butoxycarbonyl)amino]-1-hydroxy-3-phenylpropyl]oxiran

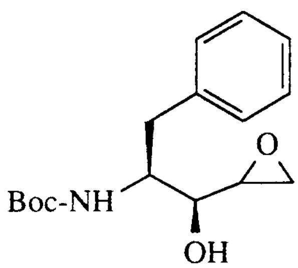

Wie für Beispiel LXIX beschrieben erhielt man aus 2,77 g (10 mmol) (3S,4S)-4-[N-(tert.Butoxycarbonyl)-amino]-3-hydroxy-5-phenylpenten [vgl. G.J. Hansen et al., J. Org. Chem. 50, 5399 (1985)] und 6,91 g (40 mmol) MCPBA nach 1 h bei 0 °C und 3,5 h bei Raumtemperatur 2,50 g (85% der Theorie) der Titelverbindung als farblose Kristalle.
Schmp.: 118 °C
$R_f$ = 0,20, II (7:3)
MS (DCI, $NH_3$): m/z = 294 $(M+H)^+$

Beispiel LXXI

2-(Trifluormethyl)-5-methyl-pyrrolidin

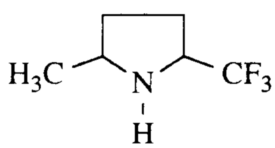

100 g (0,5 mol) 6,6,6-Trifluor-5-nitro-hexan-2-on wurden in 300 ml Methanol an 10 g Raney-Nickel bei 80 °C 8 Stunden lang in einem 0,7 l fassenden V4A-Rührautoklaven hydriert. Der Wasserstoffpartialdruck betrug 80 bar; es wurden 90% der theoretischen Menge Wasserstoff aufgenommen. Zur Aufarbeitung wurde der Katalysator abfiltriert und mit wenig Methanol nachgewaschen. Die methanolische Lösung wurde mit 100 ml

konzentrierter Salzsäure sauer gestellt und bis zu Trockne unter vermindertem Druck eingeengt. Es resultierten 59 g (0,34 mol) an 2-(Trifluormethyl)-5-methyl-pyrrolidin-Hydrochlorid (67% der Theorie). Aus dem Hydrochlorid wurde durch Zugabe einer äquimolaren Menge Natriumhydroxid-Lösung, Extraktion mit Diethylether, Trocknung über Natriumsulfat und anschließender destillativer Entfernung des Lösemittels über eine 30 cm lange Vigreux-Kolonne das 2-(Trifluormethyl)-5-methyl-pyrrolidin erhalten ($KP_{1013}$: 118-120 °C).
$^{19}F$-NMR: $\delta$ = + 1,0 ppm (d, $J_{H-F}$ = 7Hz) (gegen ext. $CF_3COOH$).

Beispiel LXXII

2-(Trifluormethyl)piperidin

a) Racemisch (Verfahrensverbesserung; M.S. Raasch, J.O.C. 27, 1406 (1972))
38,7 g (0,3 mol) D,L-Piperidin-2-carbonsäure wurden in 60 ml wasserfreiem HF und 100 g (0,925 mol) $SF_4$ für 8 h bei 120 °C in einem V4A-Rührkessel (0,3 1) umgesetzt. Anschließend wurde die HF und überschüssiges $SF_4$ im Vakkum (100 mbar) abgezogen und der Rückstand in Eis aufgenommen. Nach Filtration wurde mit 40%iger NaOH ein pH von 12 eingestellt, erneut filtriert und die wässrige Phase 4 mal mit 250 ml $Et_2O$ extrahiert. Nach Trocknen über $Na_2SO_4$ wurde der $Et_2O$ bei ND abrotiert und der Rückstand (Roh: 59 g) bei Normaldruck destilliert.
$Kp_{1013}$ (Hauptfraktion): 121-123 °C
Ausbeute: 36 g (0,235 mol) (78,4% der Theorie)
$^{19}F$-NMR: $\delta$ = + 0,45 ppm (d, $J_{HF}$ = 7 Hz) (gegen ext. $CF_3COOH$).
b) Optisch aktiv (ausgehend von L-Piperidin-2-carbonsäure)
5 g (0,039 mol) L-Piperidin-2-carbonsäure wurden analog in 30 ml HF mit 25 g (0,231 mol) $SF_4$ umgesetzt. Nach Aufarbeitung wurden 5,8 g Rohprodukt erhalten, die über eine Mikrodestille fraktioniert wurden.
$Kp_{1013}$ (Hauptfraktion): 120-122 °C
Ausbeute: 3,8 g (0,025 mol) (64,1% der Theorie)

Beispiel LXXIII

2-(Trifluormethyl)-decahydrochinolin

15 g (0,076 mol) 2-(Trifluormethyl)chinolin [hergestellt aus Chinolin-2-carbonsäure nach M.S. Raasch, J. Org. Chem. 27, 1406 (1962)] wurden in 100 ml Tetrahydrofuran an 2,5 g Ruthenium auf Aluminiumoxid (ca. 10% Ru-Gehalt) bei 180 °C 8 h lang bei 80 - 100 bar $H_2$ in einem 0,3 1 V4A-Autoklaven hydriert. Nach Filtration wurde das THF abgezogen und das Rohprodukt im Wasserstrahlvakuum übereine Mikrodestille fraktioniert.
$Kp_{16}$ (Hauptfraktion): 88 °C
Ausbeute: 12,5 g (0,061 mol) (80% der Theorie)
Das Produkt fiel als Diastereomeren-Gemisch an.
$^{19}F$-NMR: $\delta$ = + 0,65 ppm (d, $J_{H-F}$ = 7,1 Hz) (lt.Integral ca. 88%);
$^{19}F$-NMR: $\delta$ = + 0,90 ppm (d, $J_{H-F}$ = 7,1 Hz) (lt. Integral ca. 12%) (gegen ext. $CF_3COOH$).

Beispiel LXXIV

1-(Trifluormethyl)decahydroisochinolin

20 g (0,101 mol) 1-(Trifluormethyl)-3,4-dihydroisochinolin [hergestellt aus N-(Phenethyl)trifluoracetamid analog US 3 956 333 (11.5.76; Sterling Drug Inc.)] wurden in 100 ml Tetrahydrofuran an 5 g Ruthenium auf Aluminiumoxid (ca. 10% Ru-Gehalt) bei 150 °C 8 h lang bei 100 bar $H_2$ in einem 0,3 l V4A-Autoklaven hydriert. Nach Filtration wurde das THF abgezogen und das Rohprodukt im Wasserstrahlvakuum über eine Mikrodestille fraktioniert.
$Kp_{16}$ (Hauptfraktion): 94-96 °C
Ausbeute: 19 g (0,092 mol) (91% der Theorie)
Das Produkt fiel als Diastereomerengemisch an.
$^{19}F$-NMR: $\delta$ = + 5,9 ppm (d $J_{H-F}$ = 8,5 Hz) (lt. Integral ca. 99%)
$^{19}F$-NMR: $\delta$ = + 14,2 ppm (d, $J_{HF}$ = 8,5 Hz) (lt. Integral < 1%) (gegen ext. $CF_3COOH$)

Beispiel LXXV

3-(Trifluormethyl)decahydroisochinolin

Variante A

18 g (0,098 mol) Decahydroisochinolin-3-carbonsäure [hergestellt aus 1,2,3,4-Tetrahydroisochinolin-3-carbonsäure Hydrochlorid durch katalytische Hydrierung] wurden in 50 ml wasserfreiem HF mit 35 g (0,324 mol) $SF_4$ für 8 h bei 120 °C in einem V4A-Rührkessel (0,3 l) umgesetzt. Anschließend wurde die HF und überschüssiges $SF_4$ im Vakuum (100 mbar) abgezogen und der Rückstand in Eis aufgenommen. Nach Filtration wurde mit 40%iger NaOH ein pH von 12 eingestellt, erneut filtriert und die wässrige Phase 4 mal mit 250 ml $Et_2O$ extrahiert. Nach Trocknen über $Na_2SO_4$ wurde der $Et_2O$ abgezogen und der Rückstand (Roh: 9,5 g) im Wasserstrahlvakuum über eine Mikrodestille fraktioniert.
$Kp_{16}$ (Hauptfraktion): 89-94 °C
Ausbeute: 4,8 g (0,023 mol) (23,5% der Theorie)
Das Produkt fiel als Diastereomeren-Gemisch an.
$^{19}F$-NMR: $\delta$ = 1,1 ppm (d, $J_{H-F}$ = 7Hz) (lt. Integral ca.94%)
$^{19}F$-NMR: $\delta$ = -1,3 ppm (d, $J_{H-F}$ = 7 Hz) (lt. Integral ca. 6%) (gegen ext. $CF_3COOH$).

Variante B

20 g (0,101 mol) 3-(Trifluormethyl)-3,4-dihydroisochinolin [hergestellt aus N-(1,1,1-Trifluor-3-phenyl-2-propyl)formamid analog US 3 956 333 (11.5.76; Sterling Drug Inc.)] wurden in 100 ml Tetrahydrofuran an 5 g Ruthenium auf Aluminiumoxid (ca. 10% Ru-Gehalt) bei 150-180 °C 8 h lang bei 90-110 bar $H_2$ in einem 0,3 l V4A-Autoklaven hydriert. Nach Filtration wurde das THF abgezogen und das Rohprodukt im Wasserstrahlvakuum über eine Mikrodestille fraktioniert
Hauptfraktion: 93-94 °C
Ausbeute: 18,5 g (0,090 mol) (88% der Theorie)
Das Produkt fiel als Diastereomerengemisch an.

Beispiel LXXVI

(3S)-3-(N-Benzyloxycarbonyl)amino-1-chlor-4-phenyl-butan-2-on

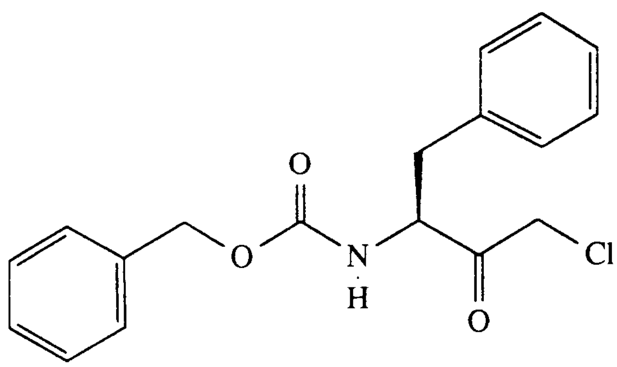

Eine Lösung aus 40 g N-Benzyloxycarbonyl-L-phenylalanin (133 mmol) in 600 ml abs. THF wird mit 23 ml abs. Triethylamin versetzt und unter Argon auf -40 °C gekühlt. Zu dieser Lösung werden 22 ml Chlorameisensäureisobutylester unter Rühren langsam zugetropft und 30 Minuten reagieren lassen. Anschließend wird der Ansatz über eine Umkehrfritte unter Kühlung vom ausgefallenen Triethylammoniumhydrochlorid befreit und in eine auf -30 °C gekühlte Lösung von 220 mmol Diazomethan in ethanolfreiem abs. Diethylether getropft. Nach 30 Minuten ist die Bildung des Diazoketons beendet. Zu dieser Lösung wird langsam eine 1 molare Lösung von wasserfreiem HCl in Diethylether bis zur bleibenden sauren Reaktion zugetropft. Nach Ende der Zugabe wird die Kühlung entfernt und noch 2 Stunden nachgerührt. Zur Aufarbeitung wird von ausgefallenen Salzen abfiltriert und das Filtrat eingeengt. Das in nahezu quantitativer Ausbeute erhaltene Produkt besitzt eine für die weitere Umsetzung hinreichende Reinheit (>90 %).

$R_f$ = 0,66 (Toluol/Aceton 6:1)

Beispiel LXXVII

(2S,3S)-3-(N-Benzyloxycarbonyl)amino-1-chlor-2-hydroxy-4-phenylbutan

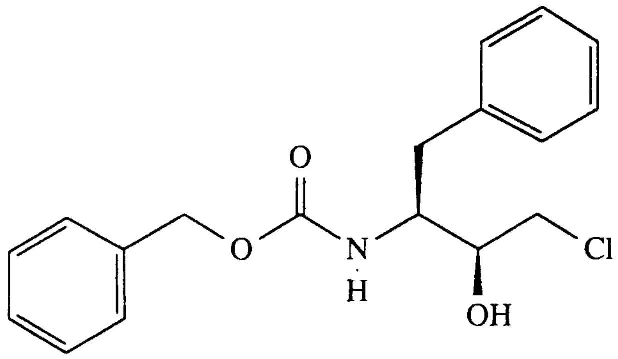

Zu einer Lösung aus 47 g der Verbindung aus Beispiel LXXVI in 500 ml Methanol werden bei 0°C 2,1 g Natriumboranat portionsweise zugegeben und noch 15 Minuten nachgerührt. Zur Aufarbeitung wird der Ansatz mit 2 n HCl auf pH6 eingestellt und eingeengt. Der erhaltene Rückstand wird in 200 ml Dichlormethan aufgenommen und zweimal mit Wasser gewaschen. Die organische Phase wird getrocknet und eingeengt. Der sirupöse Rückstand wird aus Ligroin/Aceton 4:1 kristallisiert. Nach zweimaliger Umkristallisation erhält man 20 g reines (2S)-Diastereomer. Die vereinigten Mutterlaugen werden erneut kristallisiert und liefern nach Umkristallisation weitere 5.8 g reines (2S)-Diastereomer. Das (2R)-Diastereomer kristallisiert unter diesen Bedingungen nicht.
Ausbeute [(2S)-Diastereomer]: 25,8 g (55 % der Theorie)
$R_f$ = 0,37 (Toluol/Ethanol 10:1)

Beispiel LXXVIII

(2R)-[1-(N-Benzyloxycarbonyl)amino-2-phenyl-(1S)-ethyl]oxiran

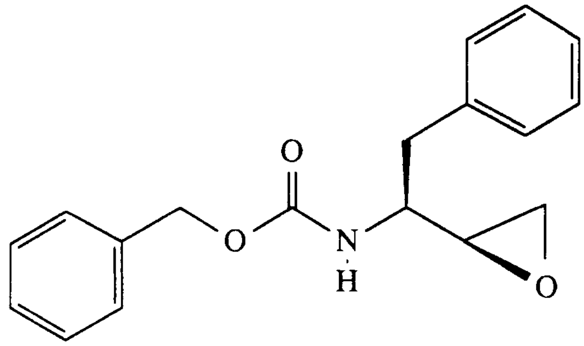

Zu einer Lösung aus 16 g der Verbindung aus Beispiel LXXVII in 600 ml Methanol werden 10 g KOH gegeben und der Ansatz bei Raumtemperatur 30 Minuten gerührt. Zur Aufarbeitung wird mit Eisessig neutralisiert und eingeengt. Der Rückstand wird in Dichlormethan aufgenommen und zweimal mit Wasser gewaschen. Die organische Phase wird getrocknet und eingeengt.
Ausbeute: 14,6 g (92 % der Theorie)
$R_f$ = 0,6 (Toluol/Ethanol 10:1)

Herstellungsbeispiele

Beispiel 1

1-[(2S und 2R, 3S)-3-[(N-tert.Butoxycarbonyl)amino]-2-hydroxy-4-phenylbutyl]-(2S)-2-(trifluormethyl)-pyrrolidin

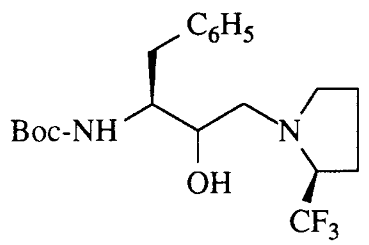

Eine Lösung von 4,00 g (15,18 mmol) (1S)-[1-[1-(tert.Butoxycarbonyl)-amino]-2-phenyl-(1S,R)-ethyl]oxiran [vgl. J.R. Luly et al., J. Org. Chem. 52, 487 (1987)] und 2,56 g (18,2 mmol) (2S)-2-(Trifluormethyl)pyrrolidin [G.V. Shustov et al., Isvest. Akad. Nauk. SSSR, 1422 (1987); engl.] in 4 ml Propanol wurde in einem Druckgefäß 2 h bei 110 °C gerührt. Nach dem Abkühlen wurde das Reaktionsgemisch im Vakuum eingeengt und nach einer Vorreinigung an 100 g Kieselgel an 600 g Kieselgel (Toluol : Ethylacetat 9:1) chromatographisch aufgetrennt. Man erhielt 3,09 g (51% der Theorie) des unpolaren Diastereomerengemisches als wachsartige Kristalle.
Schmp.: 72 °C
$R_f$ = 0,27, II (9:1)
MS (DCI, $NH_3$): m/z = 403 $(M+H)^+$
Außerdem erhielt man 1,10 g (18% der Theorie) des polaren Diastereomers als Pulver.
Schmp.: 98-100 °C
$R_f$ = 0,10, II (9:1)
MS (DCI, $NH_3$): m/z = 403 $(M+H)^+$.

Beispiel 2

1-[(2S, 3S)-3-Amino-2-hydroxy-4-phenyl-butyl]-(2S)-2-(trifluormethyl)-pyrrolidin Dihydrochlorid

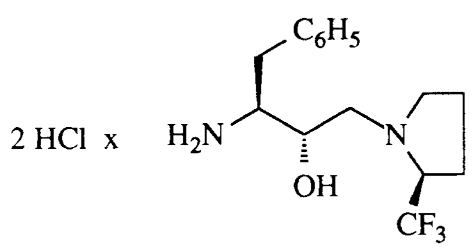

Eine Lösung von 3,09 g (7,66 mmol) des unpolaren Diastereomers aus Beispiel 1 in 30 ml einer 4 N Lösung gasförmigen Chlorwasserstoffs in wasserfreiem Dioxan wurde 30 min bei 0 °C gerührt. Danach gab man 15 ml Toluol zu und engte im Vakuum ein. Dieser Vorgang wurde noch zweimal wiederholt, dann wurde der Rückstand mit Ether verrieben, abgesaugt und im Hochvakuum über KOH getrocknet. Man erhielt 2,84 g (99% der Theorie) der Titelverbindung als farbloses Pulver.
$R_f$ = 0,42 III (9:1)
MS (FAB): m/z = 303 $(M+H)^+$, 337 $(M+Na)^+$, 359 $(M+2Na-H)^+$

Beispiel 3

1-[(2R, 3S)-3-Amino-2-hydroxy-4-phenyl-butyl]-(2S)-2(trifluormethyl)-pyrrolidin Dihydrochlorid

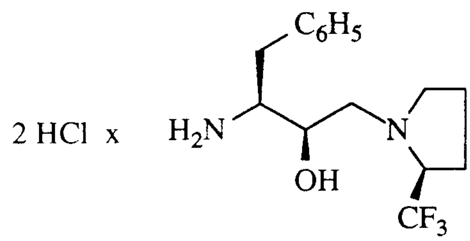

Wie für Beispiel 2 beschrieben, erhielt man aus 1,15 g (2,84 mmol) des polaren Diastereomers aus Beispiel 2 1,03 g (96% der Theorie) des polaren Amin Dihydrochlorids als Pulver.
$R_f$ = 0,40, III (9:1)
MS (DCI, $NH_3$): m/z = 303 $(M+H)^+$

Beispiel 4

1-[(2S, 3S)-3-[(N-tert.Butoxycarbonyl-L-valinyl)amino]-2-hydroxy-4-phenylbutyl]-(2S)-2-(trifluormethyl)-pyrrolidin

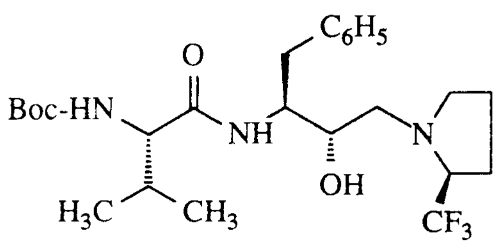

Eine auf 0 °C gekühlte, gerührte Lösung von 4,81 g (22,13 mmol) N-(tert.Butoxycarbonyl)-L-valin und 3,29 g (24,35 mmol) HOBT in 40 ml wasserfreiem Dichlormethan wurde mit 5,29 g (25,65 mmol) DCC versetzt und 5 min gerührt. Danach wurde eine Lösung von 3,70 g (20,12 mmol) der Verbindung aus Beispiel 2 und 8,85 ml (80,48 mmol) N-Methylmorpholin in 30 l Dichlormethan zugetropft. Das Kühlbad wurde entfernt und die Reaktionsmischung durfte 2 h bei Raumtemperatur rühren. Das Ende der Reaktion wurde dünnschichtchromatographisch festgestellt. Man trennte den entstandenen Harnstoff durch Filtration ab, engte das Filtrat im Vakuum ein und reinigte das Rohprodukt durch Chromatographie an 400 g Kieselgel (Dichlormethan : Methanol). Man erhielt 6,89 g (68% der Theorie) der Titelverbindung als farblosen Schaum.
MS (DCI, $NH_3$): m/z = 503 $(M+H)^+$

Beispiel 5

1-[(2R, 3S)-3-[(N-tert.Butoxycarbonyl-L-valinyl)amino]-2-hydroxy-4-phenylbutyl]-(2S)-2-(trifluormethyl)-pyrrolidin

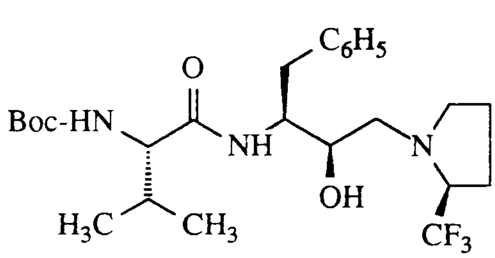

Wie für Beispiel 4 beschrieben erhielt man aus 555 mg (1,82 mmol) der Verbindung aus Beispiel 3 541 mg (59% der Theorie) des polaren Diastereomers als Schaum.
MS (FAB): m/z = 503 $(M+H)^+$

Beispiel 6

1-[(2R, 3S)-3-[(N-tert.Butoxycarbonyl-L-asparaginyl)amino]-2-hydroxy-4-phenylbutyl]-(2S)-2-(trifluormethyl)-pyrrolidin

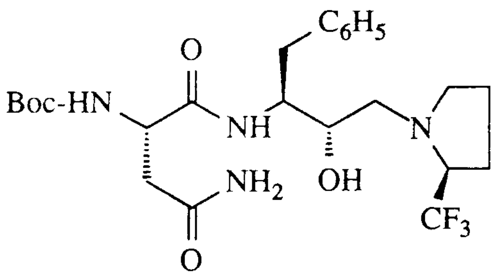

Eine gerührte Lösung von 731 mg (3,12 mmol) N-(tert.Butoxycarbonyl)-L-Asparagin in 6 ml wasserfreiem DMF wurde bei 0 °C mit 530 mg (3,44 mmol) HOBT und 1,39 g (3,28 mmol) CMTC versetzt. Danach gab man eine Lösung von 1114 mg (2,84 mmol) der Verbindung aus Beispiel 3 und 1,6 ml (14,20 mmol) N-Methylmorpholin in 8 ml DMF zu. Das Kühlbad wurde entfernt und man rührte 3 h bei Raumtemperatur. Danach wurde im Vakuum eingeengt und der Rückstand zwischen 40 ml Ethylacetat und 40 ml Wasser verteilt. Die Wasserphase wurde mit 10 ml Ethylacetat extrahiert, die vereinigten Extrakte wurden mit 50 mi Wasser gewaschen und über $MgSO_4$ getrocknet. Nach Abdampfen des Lösemittels im Vakuum und Chromatographie des Rohproduktes an 200 g Kieselgel (Dichlormethan : Methanol 9:1) erhielt man 1,42 g (89% der Theorie) der Titelverbindung als Kristalle.
Schmp.: 104 °C
$R_f$ = 0,15, I (9:1)
MS (FAB): m/z = 517 $(M+H)^+$

Beispiel 7

1-[(2S, 3S)-3-[(N-tert.Butoxycarbonyl-L-asparaginyl)amino]-2-hydroxy-4-phenylbutyl]-(2S)-2-(trifluormethyl)-pyrrolidin

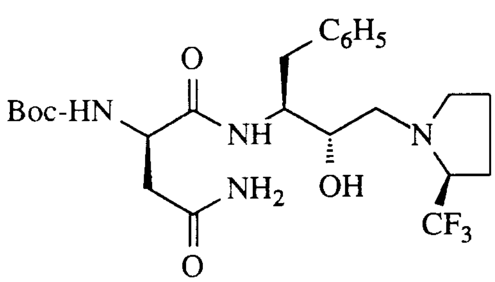

Wie für Beispiel 6 beschrieben erhielt man aus 346 mg (1,49 mmol) N-(tert.Butoxycarbonyl)-L-Asparagin und 615 mg (1,64 mmol) des unpolaren Diastereomers aus Beispiel 2 nach Chromatographie des Rohprodukts an 75 g Kieselgel (Dichlormethan: Methanol 9:1) 194 mg der Titelverbindung als Kristalle.
Schmp.: 161 °C
$R_f$ = 0,42, I (9:1)
MS (FAB): m/z = 517 $(M+H)^+$
Wie für Beispiel LXIV beschrieben erhielt man aus den entsprechenden Oxiranen (Startmaterial) und den entsprechenden Trifluormethyl-Basen $HNR^2$-$CH(CF_3)R^3$ die folgenden Produkte (Tabelle 4):
(Die Zuordnung der Stereochemie der OH-Gruppe zu den polaren bzw. unpolaren Diastereomeren der Beispiele 1-7 erfolgte tentativ.)

Tabelle 4:

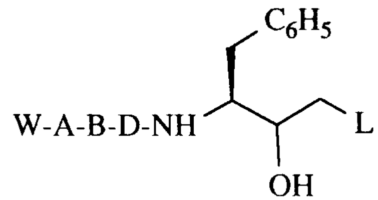

| Beispiel-Nr. | W-A-B-D- | L | Ausbeute (%) | MS(FAB) m/z $(M+H)^+$ | $R_f$/Laufmittel Verhältnis | Startmaterial aus Beispiel |
|---|---|---|---|---|---|---|
| 8 | 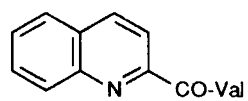 | 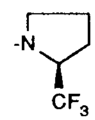 | 33 | 557 | 0,26, II (7:3) | L |
| 9 | 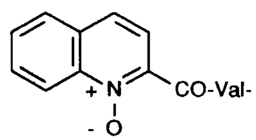 | 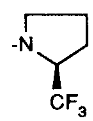 | 53 | 573 | 0,18, II (7:3) | LI |
| 10 | 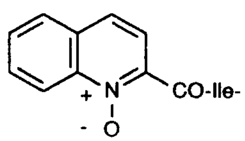 | 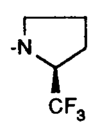 | 53 | 587 | 0,33, II (3:2) | LXIV |
| 11 | 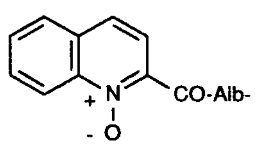 | 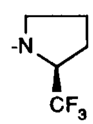 | 68 | 559 | 0,27 , I (97:3) | LXV |

Fortsetzung Tabelle 4:

| Beispiel-Nr. | W-A-B-D- | L | Ausbeute (%) | MS(FAB) m/z $(M+H)^+$ | $R_f$/Laufmittel Verhältnis F (°C) | Startmaterial aus Beispiel |
|---|---|---|---|---|---|---|
| 12 | 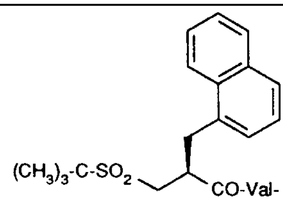 | 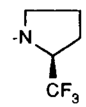 | 55 | 718 | 0,30, I (97:3) 144 | XLV |
| 13 | 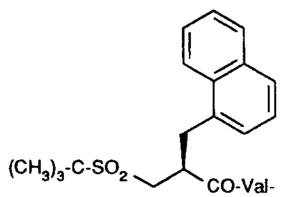 | 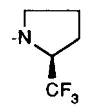 | 54 | 718 | 0,14, I (97:3) | XLIV |
| 14 | 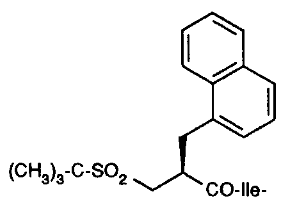 | 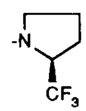 | 34 | 732 | 0,24, II (2:3) | LXVIII |
| 15 | Boc-Phe-Gly-Gly | 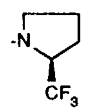 | 87 | 664 | 0,16, I (95:5) Schmp.: 172°C | LXII |

Fortsetzung Tabelle 4:

| Beispiel-Nr. | W-A-B-D- | L | Ausbeute (%) | MS(FAB) m/z $(M+H)^+$ | $R_f$/Laufmittel Verhältnis F (°C) | Startmaterial aus Beispiel |
|---|---|---|---|---|---|---|
| 16 | $CH_3(CH_2)_{12}CO$-Phe-Val- | 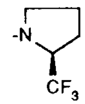 | 61 | 759 | 0,27, II (3:2) Schmp.: 109°C | LII |
| 17 | Boc-Phe-Val- | 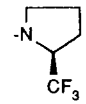 | 32 | 649 | 0,14, II (7:3) Schmp.: 155°C | XLIX |
| 18 | 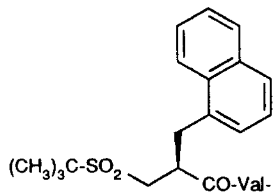 | 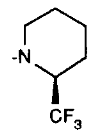 | 36 | 732 | 0,12, I (97:3) | XLV |
| 19 | 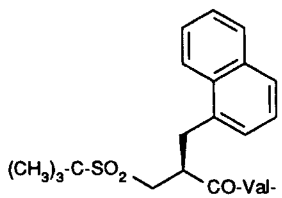 | 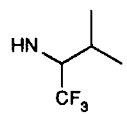 | 37 | 720 | 0,25, II (1:1) | XLV |

Fortsetzung Tabelle 4:

| Beispiel-Nr. | W-A-B-D- | L | Ausbeute (%) | MS(FAB) m/z $(M+H)^+$ | $R_f$/Laufmittel Verhältnis | Startmaterial aus Beispiel |
|---|---|---|---|---|---|---|
| 20 | 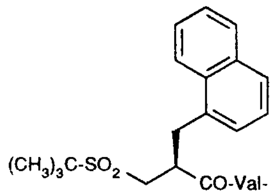 | 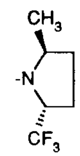 | 16 | 732 | 0,21, II (3:2) unpolar | XLV |
| 21 | 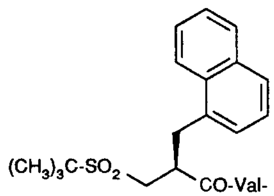 | 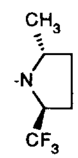 | 22 | 732 | 0,19, II (3:1) polar | XLV |
| 22 | 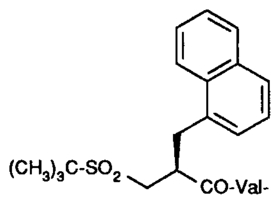 | 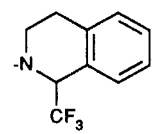 | 47 | 780 | 0,20, II (3:2) polar | XLV |
| 23 | 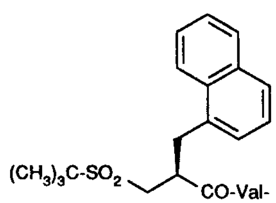 | 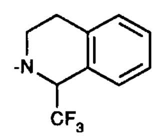 | 49 | 780 | 0,29, II (3:2) unpolar | XLIV |

Fortsetzung Tabelle 4:

| Beispiel-Nr. | W-A-B-D- | L | Ausbeute (%) | MS(FAB) m/z $(M+H)^+$ | $R_f$/Laufmittel Verhältnis | Startmaterial aus Beispiel |
|---|---|---|---|---|---|---|
| 24 | 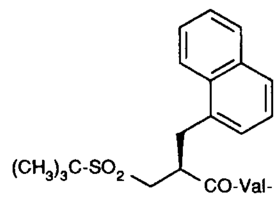 | 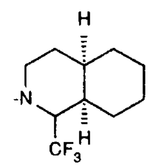 | 22 | 786 | 0,36, II (3:2) | XLV |
| 25 | 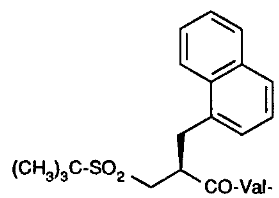 | 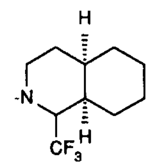 | 18 | 786 | 0,24, II (3:2) | XLV |
| 26 | 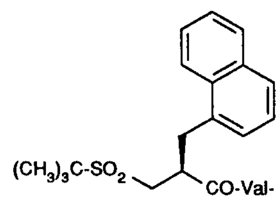 | 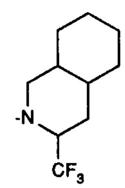 | 14 | 786 | 0,19, II (7:3) | XLV |
| 27 | 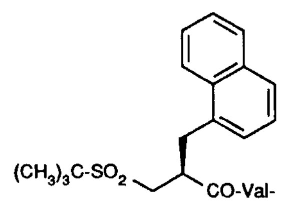 | 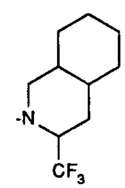 | 10 | 786 | 0,14, II (7:3) | XLV |

Fortsetzung Tabelle 4:

| Beispiel-Nr. | W-A-B-D- | L | Ausbeute (%) | MS(FAB) m/z $(M+H)^+$ | $R_f$/Laufmittel Verhältnis | Startmaterial aus Beispiel |
|---|---|---|---|---|---|---|
| 28 | 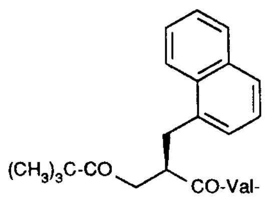 | 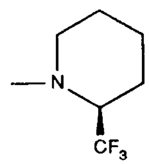 | 51 | 682 | 0,21, II (7:3) | LXVII |
| 29 | 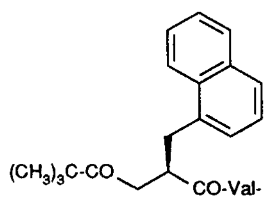 | 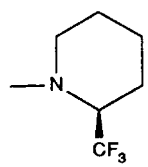 | 58 | 682 | 0,29, II (7:3) | LXVIII |

Beispiel 30

1-{(2S, 3S)-3-[(N-tert.Butoxycarbonyl-L-phenylalaninyl)-L-valinyl)amino]-4-cyclohexyl-2-hydroxybutyl}-(2S)-2-(trifluormethyl)-pyrrolidin

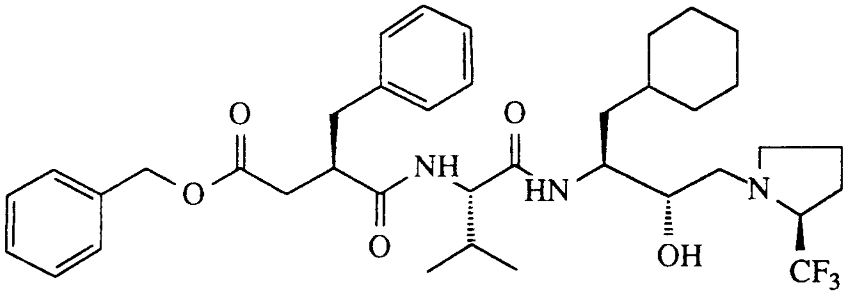

Eine Lösung von 135 mg (0,25 mmol) der Verbindung aus Beispiel LIX und 70 mg (0,50 mmol) (2S)-2-(Trifluormethyl)-pyrrolidin [vgl. G.V. Shustov et al., Isvest. Akad. Nauk. SSSR, 1422 (1987) engl.] in 0,5 ml n-Propanol wurde in einem Druckgefäß 5 h bei 110 °C gerührt. Nach dem Abkühlen wurde das Reaktiongemisch im Vakuum eingeengt und an 10 g Kieselgel (Toluol : Ethylacetat 7:3) chromatographiert. Nach Verreiben mit n-Pentan erhielt man 129 mg (76% der Theorie) der Titelverbindung als Kristalle.
Schmp.: 109 °C
$R_f$ = 0,34, II (7:3)
MS (FAB): m/z = 689 $(M+H)^+$
Wie für Beispiel 2 beschrieben erhielt man durch Abspaltung der tert.Butoxycarbonyl-Schutzgruppe aus den entsprechenden Startmaterialien die folgenden Produkte (Tabelle 5):

Tabelle 5:

2 HCl x H-A-B-D-HN 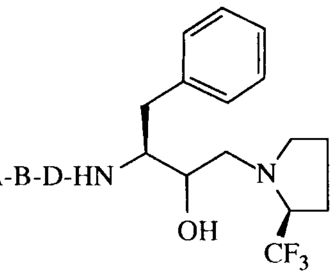

| Beispiel-Nr. | H-A-B-D- | Ausbeute (%) | MS(FAB) m/z $(M+H)^+$ | $R_f$/Laufmittel Verhältnis | Startmaterial aus Beispiel |
|---|---|---|---|---|---|
| 31 | H-Val- | 92 | 402 | 0,44, III (9:1) unpolar | 4 |
| 32 | H-Val- | 96 | 402 | 0,42, III (9:1) polar | 5 |
| 33 | H-Asn- | 94 | 417 | 0,01, I (9:1) unpolar | 7 |
| 34 | H-Asn- | 97 | 417 | 0,01, I (9:1) polar | 6 |
| 35 | H-Phe-Val- | 96 | 549 | 0,49, III (9:1) | 17 |

Wie für Beispiel 4 beschrieben, erhielt man durch Kupplung der entsprechenden Säuren mit den Aminhydrochloriden (Startmaterial) die folgenden Produkte (Tabelle 6):

Tabelle 6:

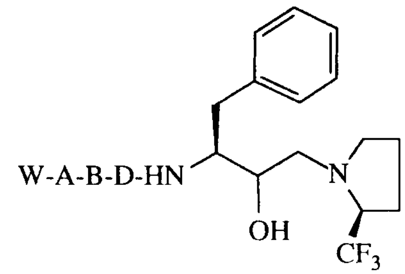

| Beispiel-Nr. | W-A-B-D- | Ausbeute (%) | MS(FAB) m/z $(M+H)^+$ | $R_f$/Laufmittel Verhältnis | Startmaterial aus Beispiel |
|---|---|---|---|---|---|
| 36 | 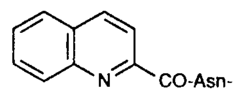 | 30 | 572 | 0,33, I (9:1) unpolar | 33 |
| 37 | 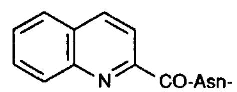 | 60 | 572 | 0,28, I (9:1) polar | 34 |
| 38 | 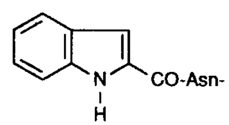 | 22 | 560 | 0,25, I (9:1) unpolar | 33 |
| 39 | 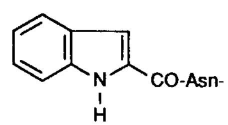 | 20 | 560 | 0,24, I (9:1) polar | 34 |

Fortsetzung Tabelle 6:

| Beispiel-Nr. | W-A-B-D- | Ausbeute (%) | MS(FAB) m/z $(M+H)^+$ | $R_f$/Laufmittel Verhältnis | Startmaterial aus Beispiel |
|---|---|---|---|---|---|
| 40 | 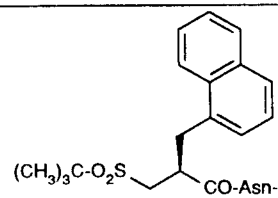 | 53 | 733 | 0,31, I (9:1) polar | 34 |
| 41 | 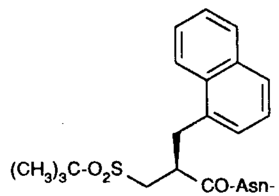 | 47 | 697 | 0,30, I (9:1) polar | 34 |
| 42 | 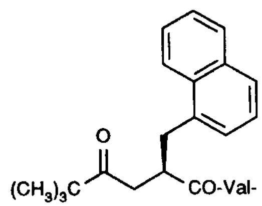 | 62 | 682 | 0,21, II (7:3) unpolar | 31 |
| 43 | 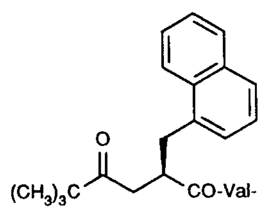 | 57 | 682 | 0,29, II (7:3) polar | 32 |

Fortsetzung Tabelle 6:

| Beispiel-Nr. | W-A-B-D- | Ausbeute (%) | MS(FAB) m/z $(M+H)^+$ | $R_f$/Laufmittel Verhältnis F (°C) | Startmaterial aus Beispiel |
|---|---|---|---|---|---|
| 44 | Boc-Phe-Val | 52 | 649 | 0,18, I (95:5) unpolar | 2 |
| 45 | Boc-Ser-Phe-Val | 45 | 736 | 0,11, I (95:5) Schmp.: 142°C | 35 |

Beispiel 46

1-{(2R und 2S, 3R, 4S)-3-[N-(tert.Butoxycarbonyl)amino]-2,3-dihydroxy-5-phenylpentyl)-(2S)-2-(trifluormethyl)pyrrolidin

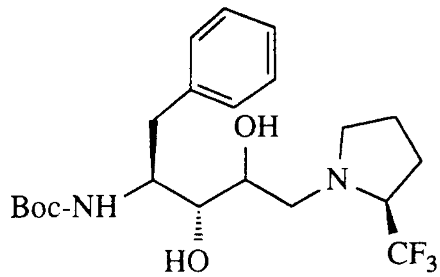

Eine Lösung von 450 mg (1,53 mmol) der Verbindung aus Beispiel LXVIII, 427 mg (3,07 mmol) (2S)-2-(Trifluormethyl)pyrrolidin [vgl. G.V. Shustov ei al., Isvest. Akad. Nauk. SSSR, 1422 (1987); engl.] in 3 ml n-Propanol wurde in einem Druckgefäß 1 h bei 110°C gerührt. Nach dem Abkühlen wurde das Reaktionsgemisch im Vakuum eingeengt und nach einer Vorreinigung an 25 g Kieselgel an 80 g Kieselgel (Toluol : Ethylacetat 7:3) chromatographisch aufgetrennt. Man erhielt 177 mg (27% der Theorie) des unpolaren Diastereomers als Pulver.
$R_f$ = 0,30, II (7:3)
MS (FAB): m/z = 433 $(M+H)^+$
Außerdem erhielt man 258 mg (39%) des polaren Diastereomers als Pulver.
$R_f$ = 0,18, I (7:3)
MS (FAB): m/z = 433 $(M+H)^+$

Beispiel 47

1-{(2R und 2S, 3S, 4S)-3-[N-(tert.Butoxycarbonyl)amino]-2,3-dihydroxy-5-phenylpentyl}-(2S)-2-(trifluormethyl)pyrrolidin

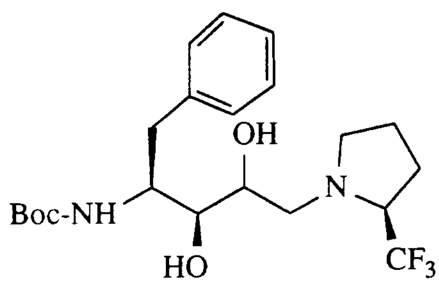

Wie für Beispiel 46 beschrieben, erhielt man aus 2,50 g (8,52 mmol) der Verbindung aus Beispiel LXIX und 2,37 g (17,04 mmol) (2S)-2-(Trifluormethyl)pyrrolidin nach Chromatographie des Rohproduktes an 442 g Kieselgel (Toluol : Acetonitril 85:15) 1,70 g (46% der Theorie) des unpolaren Diastereomers als Kristalle.
Schmp.: ab 103°C (Zers.)
$R_f$ = 0,27, II (7:3)
MS (FAB): m/z = 433 $(M+H)^+$,
sowie 923 mg (25% der Theorie) des polaren Diastereomers als Kristalle.
Schmp.: 112°C
$R_f$ = 0,19, II (7:3)
MS (FAB): m/z = 433 $(M+H)^+$

Beispiel 48

1-{(2R und 2S, 3R, 4S)-3-[N-(tert.Butoxycarbonyl)amino]-2,3-dihydroxy-5-phenylpentyl)-(2S)-2-(trifluormethyl)piperidin

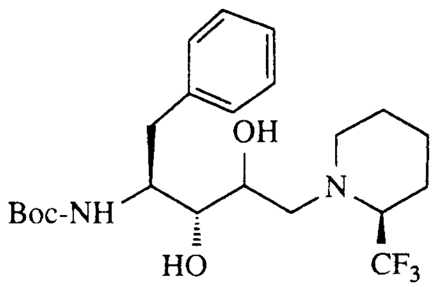

Wie für Beispiel 46 beschrieben, erhielt man aus 690 mg (2,35 mmol) der Verbindung aus Beispiel LXVIII und 720 mg (4,70 mmol) (2S)-2-(Trifluormethyl)piperidin nach Chromatographie des Rohproduktes an 75 g Kieselgel (Toluol : Acetonitril 9:1) 218 mg (21% der Theorie) des unpolaren Diastereomers als Sirup.

$R_f$ = 0,26, V (9:1)

MS (FAB): m/z = 447 $(M+H)^+$

Sowie 351 mg (33%) des polaren Diasteromers als Sirup.

$R_f$ = 0,06, V (9:1)

MS (FAB): m/z = 447 $(M+H)^+$

Wie für Beispiel 2 beschrieben, erhielt man durch Abspaltung der tert.Butoxycarbonyl-Schutzgruppe aus den entsprechenden Startmaterialien die folgenden Aminhydrochloride (Tabelle 7):

Tabelle 7

2 HCl x 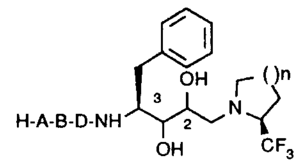

| Beispiel-Nr. | H-A-B-D- | n | 3-(OH)-Stereochemie | Ausbeute (%) | MS(FAB) m/z $(M+H)^+$ | $R_f$/Laufmittel Verhältnis | Startmaterial aus Beispiel / (Isomer) |
|---|---|---|---|---|---|---|---|
| 48 | H- | 1 | R | 97 | 333 | 0,20, III (95:5) | 46 (unpolar) |
| 49 | H- | 1 | R | 96 | 333 | 0,20, III (95:5) | 46 (polar) |
| 50 | H- | 1 | S | 98 | 333 | 0,42, III (9:1) | 47 (unpolar) |
| 51 | H- | 1 | S | 94 | 333 | 0,41, III (9:1) | 47 (polar) |
| 52 | H- | 2 | R | 97 | 347 | 0,26, III (95:5) | 48 (unpolar) |
| 53 | H- | 2 | R | 95 | 347 | 0,24, III (95:5) | 48 (polar) |
| 54 | H-Val | 1 | R | 96 | 432 | 0,30 , III (95:5) | 60 (unpolar) |
| 55 | H-Val | 1 | R | 98 | 432 | 0,29, III (95:5) | 60 (polar) |

Fortsetzung Tabelle 7

| Beispiel-Nr. | H-A-B-D- | n | 3-(OH)-Stereochemie | Ausbeute (%) | MS(FAB) m/z $(M+H)^+$ | $R_f$/Laufmittel Verhältnis | Startmaterial aus Beispiel (Isomer) |
|---|---|---|---|---|---|---|---|
| 56 | H-Val | 2 | R | 97 | 347 | 0,32, III (95:5) | 62 (unpolar) |
| 57 | H-Val | 2 | R | 99 | 347 | 0,30, III (95:5) | 63 (polar) |
| 58 | H-Asn | 1 | S | 96 | 447 | 0,30, III (9:1) | 65 (unpolar) |
| 59 | H-Asn | 1 | S | 98 | 447 | 0,27, III (9:1) | 67 (polar) |

Wie für Beispiel 4 bzw. 6 beschrieben, erhielt man durch Kupplung der entsprechenden Säuren mit den Aminhydrochloriden (Startmaterial) die folgenden Produkte (Tabelle 8):

Tabelle 8

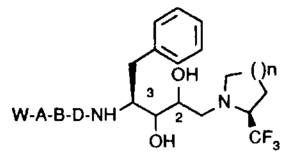

| Beispiel-Nr. | W-A-B-D- | n | 3-(OH)-Stereochemie | Ausbeute (%) | MS(FAB) m/z $(M+H)^+$ | $R_f$/Laufmittel Verhältnis Schmp. [°C] | Startmaterial aus Beispiel |
|---|---|---|---|---|---|---|---|
| 60 | Boc-Val | 1 | R | 81 | 532 | 0,27, III (3:2) amorph | 48 |
| 61 | Boc-Val | 1 | R | 56 | 532 | 0,22, III (3:2) amorph | 49 |
| 62 | Boc-Val | 2 | R | 74 | 546 | 0,25, III (7:3) amorph | 52 |
| 63 | Boc-Val | 2 | R | 63 | 546 | 0,28, III (3:2) amorph | 53 |
| 64 | 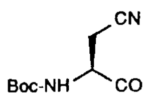 | 1 | S | 8 | 529 | 0,38, I (95:5) 181 | 50 |
| 65 | Boc-Asn | 1 | S | 43 | 547 | 0,14, I (95:5) 212 | |
| 66 | 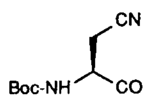 | 1 | S | 9 | 529 | 0,26, I (95:5) 173 | 51 |
| 67 | 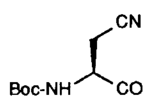 | 1 | S | 36 | 547 | 0,11, I (95:5) 145 | |

Fortsetzung Tabelle 8

| Beispiel-Nr. | W-A-B-D- | n | 3-(OH)-Stereochemie | Ausbeute (%) | MS(FAB) m/z $(M+H)^+$ | $R_f$/Laufmittel Verhältnis Schmp. [°C] | Startmaterial aus Beispiel |
|---|---|---|---|---|---|---|---|
| 68 | 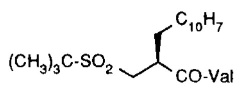 | 1 | R | 84 | 748 | 0,28, II (2:3) 163 | 54 |
| 69 | 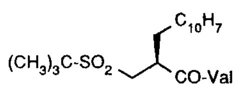 | 1 | R | 52 | 748 | 0,19, II (2:3) ab 97 (Zers.) | 55 |
| 70 | 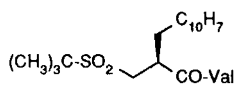 | 2 | R | 72 | 762 | 0,15, I (97:3) 155 | 62 |
| 71 | 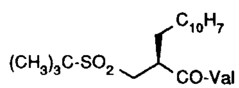 | 2 | R | 48 | 762 | 0,06, I (97:3) ab 88 (Zes.) | 63 |
| 72 | 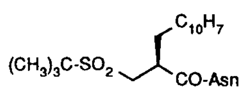 | 1 | S | 62 | 763 | 0,22, I (97:3) 118 | 58 |
| 73 | 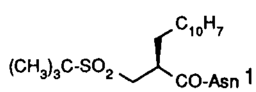 | 1 | S | 52 | 763 | 0,15, I (97:3) 110 | 59 |

Fortsetzung Tabelle 8

| Beispiel-Nr. | W-A-B-D- | n | 3-(OH)-Stereochemie | Ausbeute (%) | MS(FAB) m/z $(M+H)^+$ | $R_f$/Laufmittel Verhältnis Schmp. [°C] | Startmaterial aus Beispiel |
|---|---|---|---|---|---|---|---|
| 74 | 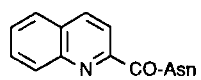 | 1 | S | 72 | 602 | 0,15, I (95:5) 190 | 58 |
| 75 | 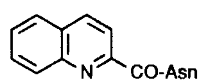 | 1 | S | 52 | 602 | 0,10, I (95:5) 137 | 59 |

Beispiel 76

1-{(2R,3S)-3-[(N-benzyloxycarbonyl)amino]-2-hydroxy-4-phenyl-butyl}-2-(trifluormethyl)pyrrolidon

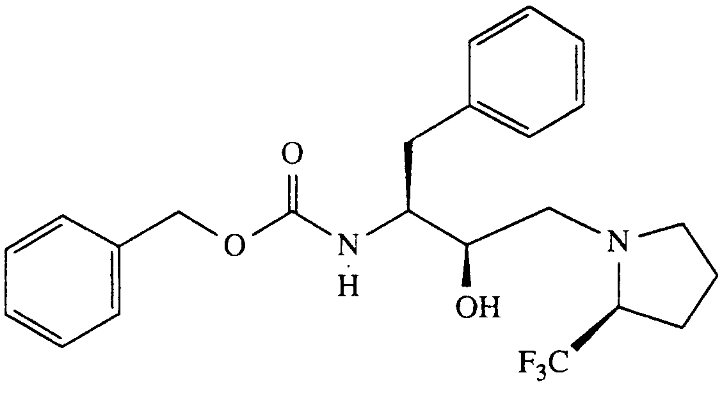

2 g (2S)-2-(trifluormethyl)pyrrolidin (14,4 mmol) und 4 g der Verbindung aus Beispiel LXXVIII (14,3 mmol) werden in 8 ml 2-Propanol gelöst und in einem Druckrohr bei 100°C gerührt. Nach 4 Stunden ist die Umsetzung beendet. Zur Aufarbeitung wird mit 20 ml Dichlormethan verdünnt, zweimal mit Wasser gewaschen. Die organische Phase wird getrocknet und eingeengt. Anschließende chromatographische Reinigung (Laufmittel: Cyclohexan/Aceton 3:1) ergibt 1,54 g der Titelverbindung.
Ausbeute: 1,54 g (25 % der Theorie)
$R_f$ = 0,24 (Cyclohexan/Aceton 3:1)

Beispiel 77

1-{(2R,3S)-3-Amino-2-hydroxy-4-phenylbutyl]-(2S)-2-(trifluormethyl)pyrrolidin

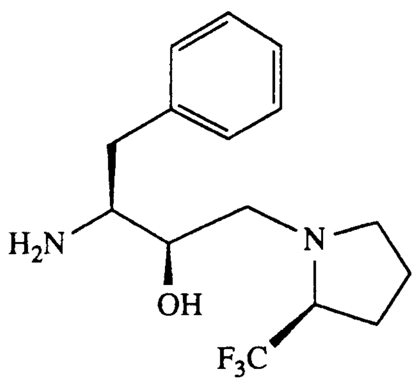

Eine Lösung von 1,5 g der Verbindung aus Beispiel 76 (3,5 mmol) in 10 ml Methanol wird mit 200 mg Pd-C (10 %) versetzt und unter Normaldruck bei Raumtemperatur hydriert. Zur Aufarbeitung wird vom Katalysator abfiltriert und eingeengt.
Ausbeute: 1,0 g (96 % der Theorie)
$R_f$ = 0,2 (Toluol/Ethanol 3:1)

Beispiel 78

1-{(2R,3S)-3-[(N-Benzyloxycarbonyl-L-asparaginyl)amino]-2-hydroxy-4-phenylbutyl}-(2S)-2-(trifluormethyl)-pyrrolidin

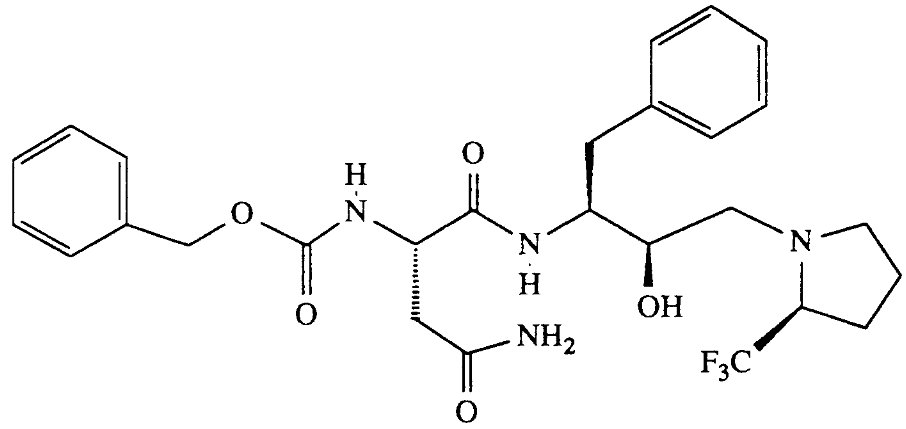

Wie für Beispiel 6 beschrieben, erhielt man aus der Verbindung aus Beispiel 77 (3,6 mmol) 800 mg der Titelverbindung nach chromatographischer Reinigung des Rohproduktes (Laufmittel: Dichlormethan/Methanol 9:1).
Ausbeute: 800 mg (40 % der Theorie)
$R_f$ = 0,3 (Dichlormethan/Methanol 9:1)
MS (FAB): m/z = 551 $(M+H)^+$

Beispiel 79

1-{(2R,3S)-3-[(L-asparaginyl)amino]-2-hydroxy-4-phenylbutyl}-(2S)-2-(trifluormethyl)pyrrolidin

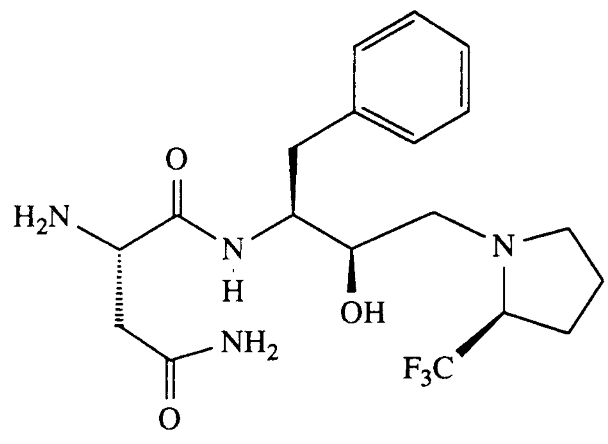

Wie für Beispiel 77 beschrieben, erhielt man aus 700 mg der Verbindung aus Beispiel 78 (1,3 mmol) 448 mg der Titelverbindung nach chromatographischer Reinigung des Rohproduktes (Laufmittel: Dichlormethan/Methanol 9:1).
Ausbeute: 448 mg (83 % der Theorie)
$R_f$ = 0,36 (Dichlormethan/Methanol 9:1)
MS (FAB): m/z = 417 $(M+H)^+$

Beispiel 80

1-{(2R,3S)-3-[(N-Isochinolin-2-carbonyl-L-asparaginyl)amino]-2-hydroxy-4-phenylbutyl}-(2S)-2-(trifluormethyl)pyrrolidin

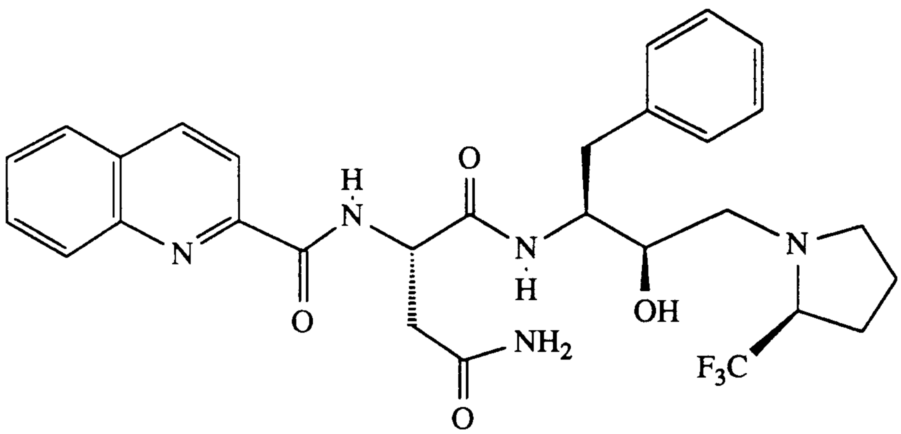

Wie für Beispiel 4 beschrieben, erhielt man aus 440 mg der Verbindung aus Beispiel 79 (1,1 mmol) 390 mg der Titelverbindung nach chromatographischer Reinigung des Rohproduktes (Laufmittel: Toluol/Ethanol 10:1).
Ausbeute: 339 mg (64 % der Theorie)
$R_f$ = 0,15 (Toluol/Ethanol 9:1)
MS (FAB): m/z = 572 $(M+H)^+$

Beispiel 81

2-{(2R,3S)-3-[(N-Benzyloxycarbonyl)amino]-2-hydroxy-4-phenyl-butyl}-(1S,4aR,8aR)-1-(trifluormethyl)-decahydroisochinolin

und

Beispiel 82

2-{(2R,3S)-3-[(N-Benzyloxycarbonyl)amino]-2-hydroxy-4-phenyl-butyl}-(1R,4aS,8aS)-1-(trifluormethyl)-decahydroisochinolin

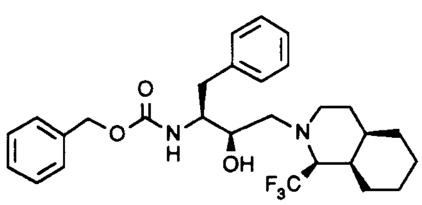

+

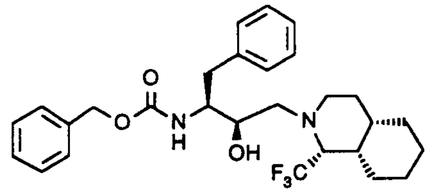

(81) (82)

1,25 g des 1:1 Enantiomerenpaares (1S,4aR,8aR)-1(trifluormethyl)-decahydroisochinolin und (1R,4aS,8aS)-1-(trifluormethyl)-decahydroisochinolin (6,0 mmol) und 1,7 g der Verbindung aus Beispiel LXXVIII (6,0 mmol) wurden analog zu Beispiel 76 umgesetzt. Anschließende chromatographische Trennung der Diastereomeren (Laufmittel: Toluol/Ethylacetat 5:1) ergab 430 mg Beispiel 81 und 390 mg Beispiel 82.
Ausbeute: 430 mg Beispiel 81 (15 % der Theorie)
$R_f$ = 0,56 (Toluol/Ethanol 10:1)
Ausbeute: 390 mg Beispiel 82 (13 % der Theorie)
$R_f$ = 0,42 (Toluol/Ethanol 10:1)

Beispiel 83

2-[(2R,3S)-3-Amino-2-hydroxy-4-phenylbutyl]-(1S,4aR,8aR)-1-(trifluormethyl)decahydroisochinolin

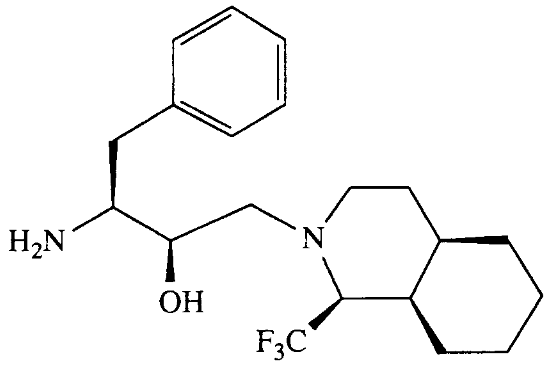

Wie für Beispiel 77 beschrieben, erhielt man durch Hydrierung aus 400 mg der Verbindung aus Beispiel 81 277 mg der Titelverbindung.
Ausbeute: 277 mg (94 % der Theorie)
$R_f$ = 0,24 (Toluol/Ethanol 10:1)

Beispiel 84

2-[(2R,3S)-3-Amino-2-hydroxy-4-phenylbutyl]-(1R,4aS,8aS)-1-(trifluormethyl)decahydroisochinolin

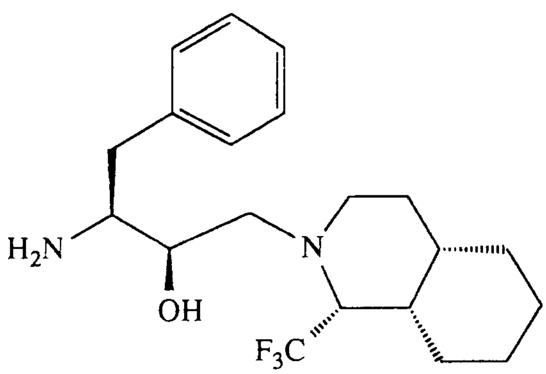

Wie für Beispiel 77 beschrieben, erhielt man durch Hydrierung aus 360 mg Verbindung aus Beispiel 82 249 mg der Titelverbindung.
Ausbeute: 249 mg (94 % der Theorie)
$R_f$ = 0,19 (Toluol/Ethanol 10:1)

Beispiel 85

2-{(2R,3S)-3-[(N-Benzyloxycarbonyl-L-asparaginyl)amino]-2-hydroxy-4-phenylbutyl}-(1S,4aR,8aR)-1-(trifluormethyl)-decahydroisochinolin

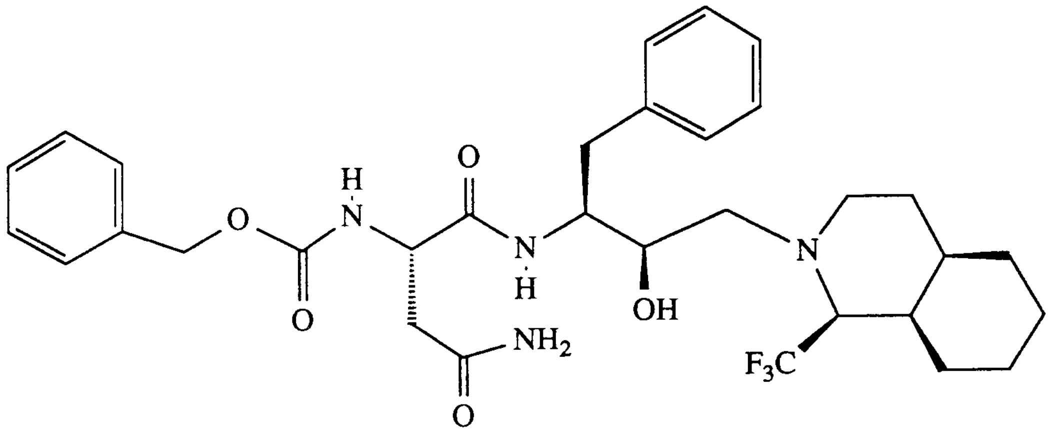

Wie für Beispiel 6 beschrieben, erhielt man aus 380 mg der Verbindung aus Beispiel 83 (1,0 mmol) 275 mg der Titelverbindung nach chromatographischer Reinigung des Rohproduktes (Laufmittel: Toluol/Ethanol 10:1)
Ausbeute: 275 mg (43 % der Theorie)
$R_f$ = 0,16 (Toluol/Ethanol 10:1)
MS (FAB): m/z = 619 $(M+H)^+$

Beispiel 86

2-{(2R,3S)-3-[(N-Benzyloxycarbonyl-L-asparaginyl)amino]-2-hydroxy-4-phenylbutyl}-(1R,4aS,8aS)-1-(trifluormethyl)-decahydroisochinolin

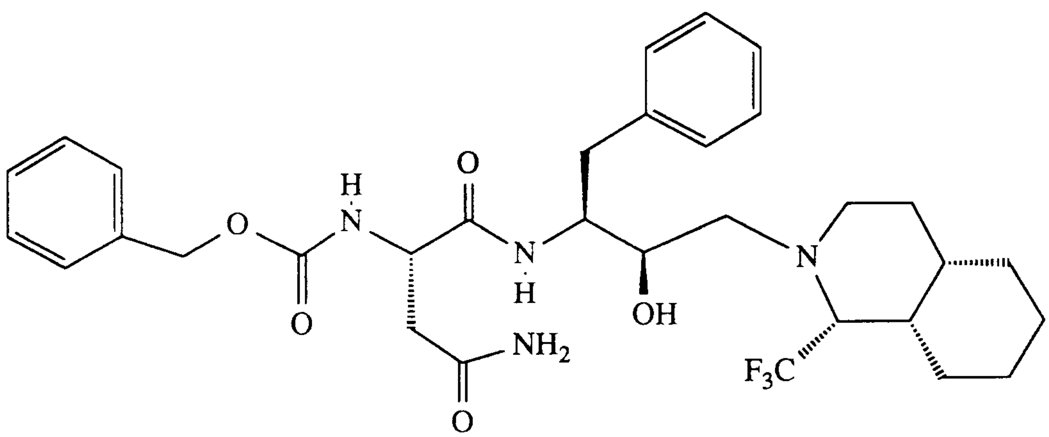

Wie für Beispiel 6 beschrieben, erhielt man aus 220 mg der Verbindung aus Beispiel 83 (0,6 mmol) 130 mg der Titelverbindung nach chromatographischer Reinigung des Rohproduktes (Laufmittel: Toluol/Ethanol 10:1).
Ausbeute: 130 mg (37 % der Theorie)
$R_f$ = 0,13 (Toluol/Ethanol 10:1)
MS (FAB): m/z = 619 $(M+H)^+$

Beispiel 87

2-{(2R,3S)-3-[(L-asparaginyl)amino]-2-hydroxy-4-phenylbutyl]-(1S,4aR,8aR)-1-(trifluormethyl)-decahydroisochinolin

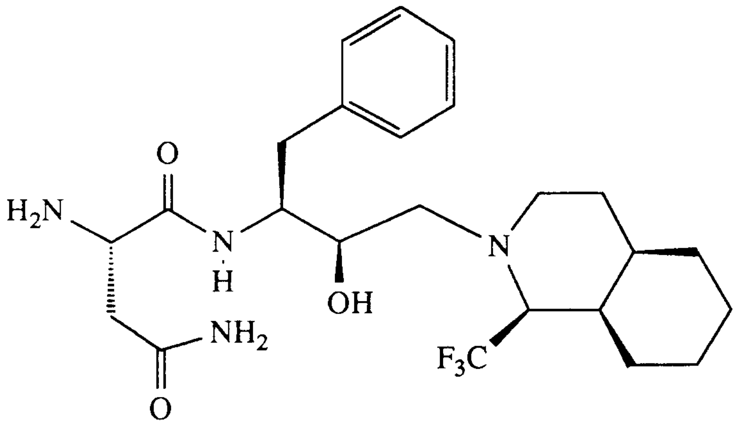

Wie für Beispiel 77 beschrieben, erhielt man aus 260 mg der Verbindung aus Beispiel 85 (0,4 mmol) 200 mg der Titelverbindung.
Ausbeute: 200 mg (98 % der Theorie)
$R_f$ = 0,38 (Dichlormethan/Methanol 9:1)
MS (FAB): m/z = 485 $(M+H)^+$

Beispiel 88

2-{(2R,3S)-3-[(L-asparaginyl)amino]-2-hydroxy-4-phenylbutyl]-(1R,4aS,8aS)-1-(trifluormethyl)-decahydroisochinolin

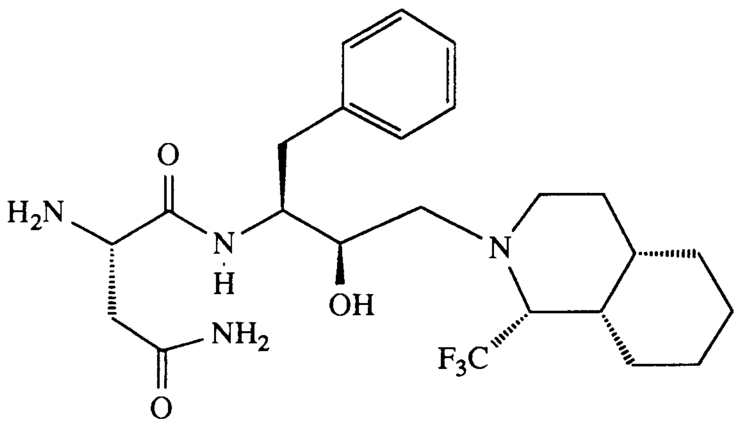

Wie für Beispiel 77 beschrieben, erhielt man aus 190 mg der Verbindung aus Beispiel 86 (0,3 mmol) 132 mg der Titelverbindung.
Ausbeute: 132 mg (98 % der Theorie)
$R_f$ = 0,31 (Dichlormethan/Methanol 9:1)
MS (FAB): m/z = 485 $(M+H)^+$

Beispiel 89

2-{(2R,3S)-3-[(N-Isochinolin-2-carbonyl-L-asparaginyl)amino]-2-hydroxy-4-phenylbutyl}-(1S,4aR,8aR)-1-(trifluormethyl)-decahydroisochinolin

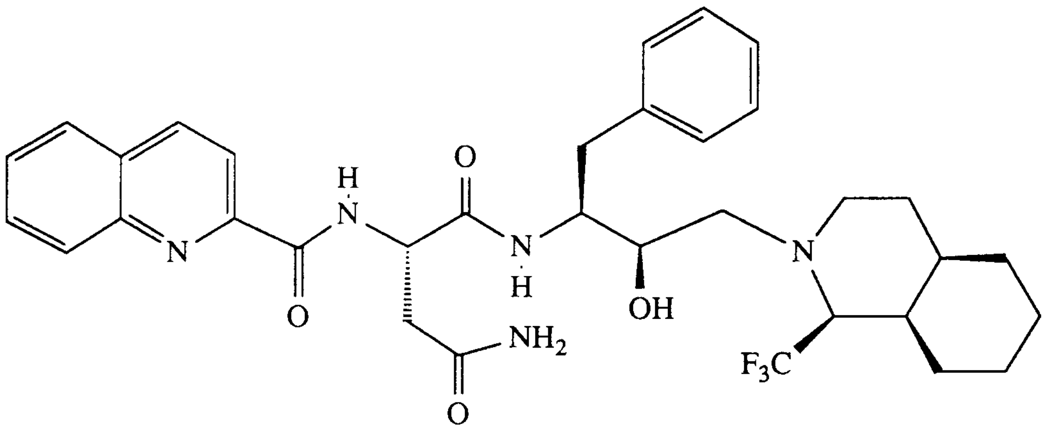

Wie für Beispiel 4 beschrieben, erhielt man aus 180 mg der Verbindung aus Beispiel 87 (372 µmol) 133 mg der Titelverbindung nach chromatographischer Reinigung des Rohproduktes (Laufmittel: Toluol/Ethanol 3:1).
Ausbeute: 133 mg (56 % der Theorie)
$R_f$ = 0,48 (Toluol/Ethanol 3:1)
MS (FAB): m/z = 640 $(M+H)^+$

Beispiel 90

2-{(2R,3S)-3-[(N-Isochinolin-2-carbonyl-L-asparaginyl)amino]-2-hydroxy-4-phenylbutyl}-(1R,4aS,8aS)-1-(trifluormethyl)-decahydroisochinolin

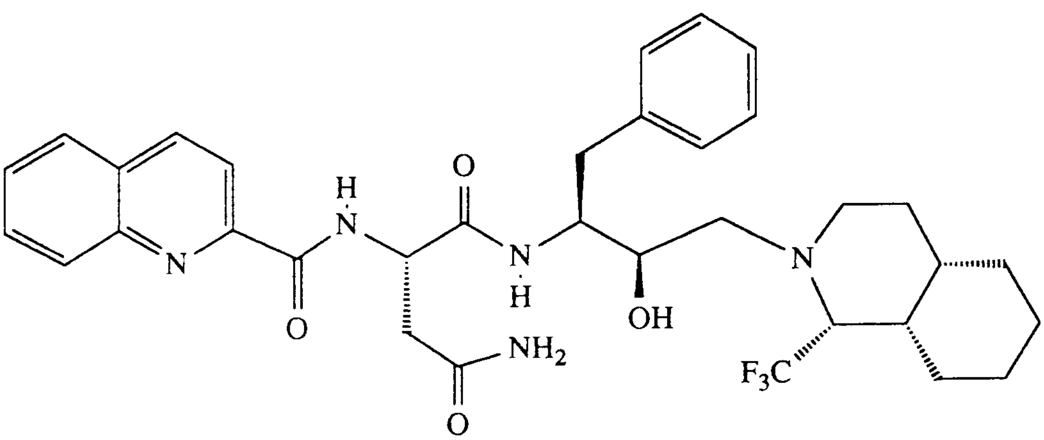

Wie für Beispiel 4 beschrieben, erhielt man aus 110 mg der Verbindung aus Beispiel 88 (227 µmol) 69 mg der Titelverbindung nach chromatographischer Reinigung des Rohproduktes (Laufmittel: Toluol/Ethanol 3:1).
Ausbeute: 69 mg (48 % der Theorie)
$R_f$ = 0,41 (Toluol/Ethanol 3:1)
MS (FAB): m/z = 640 $(M+H)^+$

Beispiel 91

1-{(2R,3S)-3-[(N-Benzyloxycarbonyl)amino]-2-hydroxy-4-phenylbutyl}-(2S,5R)-5-methyl-2-(trifluormethyl)-pyrrolidin

und

Beispiel 92

1-{(2R,3S)-3-[(N-Benzyloxycarbonyl)amino]-2-hydroxy-4-phenylbutyl}-(2R,5S)-5-methyl-2-(trifluormethyl)-pyrrolidin

(91)

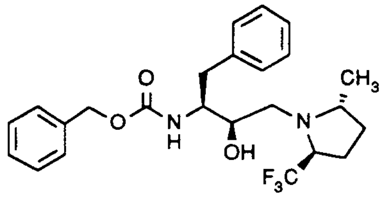

+

(92)

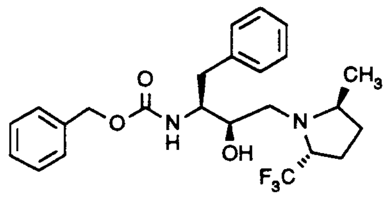

2 g des 1:1 Enantiomerenpaares (2S,5R)-5-methyl-2-(trifluormethyl)-pyrrolidin und (2S,5R)-5-methyl-2-(trifluormethyl)-pyrrolidin (13,1 mmol) und 3,66 g der Verbindung aus Beispiel LXXVIII (13,1 mmol) wurden analog zu Beispiel 76 umgesetzt. Anschließende chromatographische Reinigung (Laufmittel: Cyclohexan/Aceton 3:1) ergab 2,1 g des Diastereomerenpaares der Titelverbindungen.

Ausbeute: 2,1 g (36% der Theorie)

$R_f$ = 0,52 (Toluol/Ethanol 10:1)

Beispiel 93

1-{(2R,3S)-3-Amino-2-hydroxy-4-phenylbutyl}-(2S,5R)-5-methyl-2-(trifluormethyl)-pyrrolidin

und

Beispiel 94

1-{(2R,3S)-3-Amino-2-hydroxy-4-phenylbutyl}-(2R,5S)-5-methyl-2-(trifluormethyl)-pyrrolidin

(93)

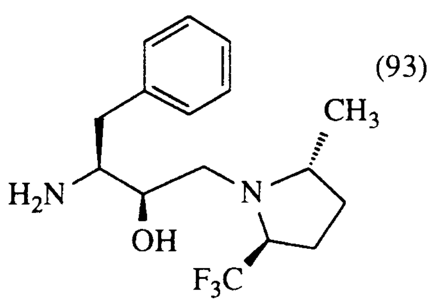

+

(94)

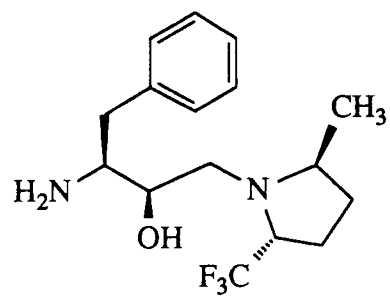

2 g des Diastereomerenpaares der Verbindungen 91 und 92 (4,4 mmol) wurden analog zu Beispiel 77 hydriert.

Ausbeute: 1,38 g (98 % der Theorie)

$R_f$ = 0,20 (Toluol/Ethanol 10:1)

Beispiel 95

1-{(2R,3S)-3-[(N-Benzyloxycarbonyl-L-asparaginyl)amino]-2-hydroxy-4-phenylbutyl}-(2S,5R)-5-methyl-2-(trifluormethyl)pyrrolidin

und

Beispiel 96 1-{(2R,3S)-3-[(N-Benzyloxycarbonyl-L-asparaginyl)amino]-2-hydroxy-4-phenylbutyl)-(2R,5S)-5-methyl-2-(trifluormethyl)pyrrolidin

(95)

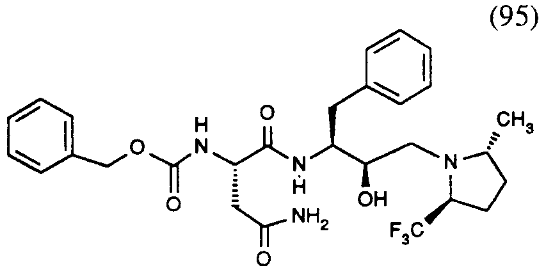

+

(96)

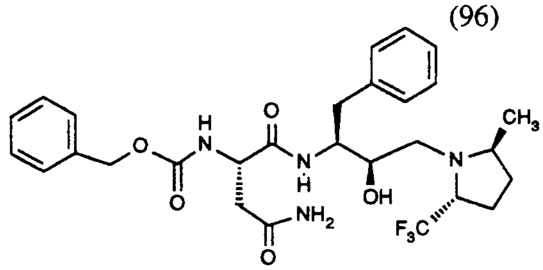

Wie für Beispiel 6 beschrieben, erhielt man aus 1,38 g der Verbindungen aus Beispiel 93 und 94 (4,4 mmol) 1,08 g der Titelverbindung nach chromatographischer Reinigung des Rohproduktes (Laufmittel: Toluol/Ethanol 10:1).
Ausbeute: 1,08 g (44 % der Theorie)
$R_f$ = 0,13 (Toluol/Ehanol 10:1)
MS (FAB): m/z = 565 $(M+H)^+$

Beispiel 97

1-{(2R,3S)-3-[(L-asparaginyl)amino]-2-hydroxy-4-phenylbutyl)-(2S,5R)-5-methyl-2-(trifluormethyl)pyrrolidin und

Beispiel 98

1-{(2R,3S)-3-[(L-asparaginyl)amino-2-hydroxy-4-phenylbutyl}-(2R,5S)-5-methyl-2-(trifluormethyl)pyrrolidin

(97)

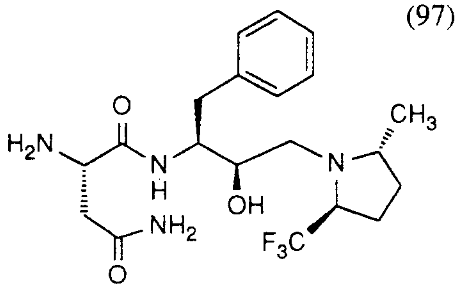

+

(98)

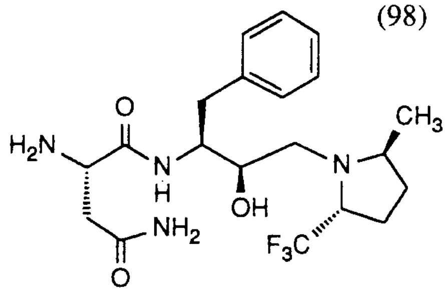

Wie für Beispiel 77 beschrieben, erhielt man aus 980 mg der Verbindungen 95 und 96 (1,7 mmol) 725 mg der Titelverbindung.
Ausbeute: 725 mg (99 % der Theorie)
$R_f$ =0,32 (Dichlormethan/Methanol 9:1)
MS (FAB): m/z = 431 $(M+H)^+$

Beispiel 99

1-{(2R,3S)-3-[(N-Isochinolin-2-carbonyl-L-asparaginyl)amino]-2-hydroxy-4-phenylbutyl}-(2S,5R)-5-methyl-2-(trifluormethyl)pyrrolidin und

Beispiel 100

1-{(2R,3S)-3-[(N-Isochinolin-2-carbonyl-L-asparaginyl)amino]-2-hydroxy-4-phenylbutyl}-(2R,5S)-5-methyl-2-(trifluormethyl)pyrrolidin

(99)

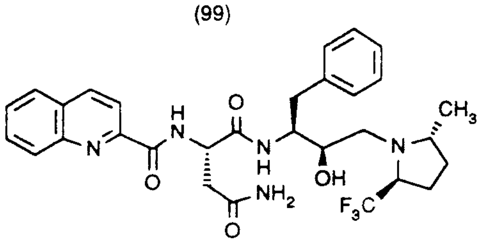

+

(100)

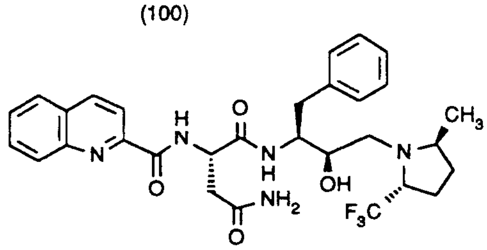

Wie für Beispiel 4 beschrieben, erhielt man aus 729 mg der Verbindungen 97 und 98 (1,7 mmol) 700 mg der Titelverbindung nach chromatographischer Reinigung des Rohproduktes (Laufmittel: Toluol/Ethanol 10:1).
Ausbeute: 700 mg (70 % der Theorie)
$R_f$ = 0,18 (Toluol/Ethanol 10:1)
MS (FAB): m/z = 586 $(M+H)^+$

**Patentansprüche**

**1.** Verbindungen der allgemeinen Formel (I)

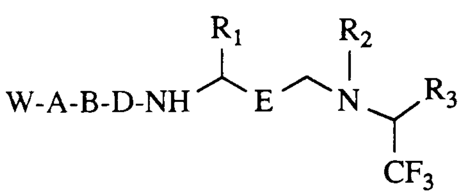

(I)

in welcher

W für Wasserstoff oder für eine typische Aminoschutzgruppe steht, oder
für geradkettiges oder verzweigtes Alkyl oder Alkenyl mit jeweils bis zu 6 Kohlenstoffatomen steht, die gegebenenfalls durch Aryl mit 6 bis 10 Kohlenstoffatomen substituiert sind oder
für eine Gruppe der Formel $R^4$-CO-, $R^5R^6$N-CO- oder $R^7$-$SO_2$- steht,

worin

$R^4$ Wasserstoff, Trifluormethyl oder geradkettiges oder verzweigtes Alkoxy mit bis zu 8 Kohlenstoffatomen oder Alkyl mit bis zu 18 Kohlenstoffatomen bedeutet, das gegebenenfalls bis zu 2-fach gleich oder verschieden durch Aryl mit 6 bis 10 Kohlenstoffatomen oder Pyridyl substituiert ist, oder
Aryl mit 6 bis 10 Kohlenstoffatomen bedeutet, das gegebenenfalls durch Halogen, Trifluormethyl, Trifluormethoxy oder durch geradkettiges oder verzweigtes Alkyl mit bis zu 8 Kohlenstoffatomen substituiert ist,
Cycloalkyl mit 3 bis 7 Kohlenstoffatomen bedeutet, oder
Chinolyl, Chinolyl-N-oxid, Indolyl, Pyridyl, Morpholino oder Piperazinyl bedeutet, oder
einen Rest der Formel

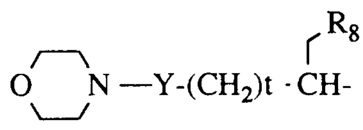 ,

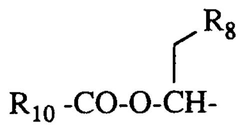 ,

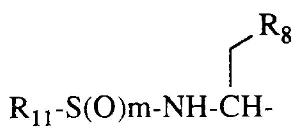 ,

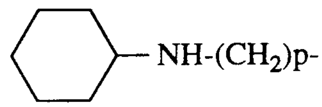 ,

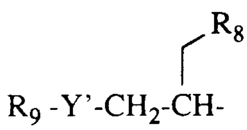

oder

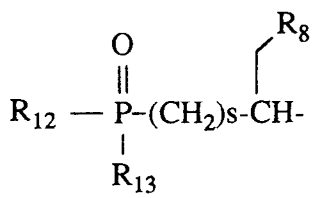

bedeutet,
worin

$R^8$ Phenyl oder Naphthyl bedeutet,

$R^9$ und $R^{10}$ unabhängig voneinander geradkettiges oder verzweigtes Alkyl mit bis zu 14 Kohlenstoffatomen bedeuten, das gegebenenfalls durch Phenyl oder Naphthyl substituiert ist, oder Benzyloxy oder Aryl mit 6 bis 10 Kohlenstoffatomen bedeuten, welches seinerseits durch Alkyl mit bis zu 4 Kohlenstoffatomen substituiert ist,

$R^{11}$ die oben angegebene Bedeutung von $R^9$ und $R^{10}$ hat und mit dieser gleich oder verschieden ist oder über N-gebundenes Morpholino oder Pyrrolidinyl bedeutet,

m eine Zahl 0, 1 oder 2 bedeutet,

p eine Zahl 1, 2 oder 3 bedeutet,

Y und Y' unabhängig voneinander für CO- oder $SO_2$- stehen,

t eine Zahl 0,1 oder 2 bedeutet,

$R^{12}$ und $R^{13}$ unabhängig voneinander Hydroxy oder Alkoxy mit bis zu 8 Kohlenstoffatomen bedeuten,

s eine Zahl 1 oder 2 bedeutet,

$R^5$ und $R^6$ unabhängig voneinander Wasserstoff oder
Aryl mit 6 bis 10 Kohlenstoffatomen bedeuten, das gegebenenfalls durch geradkettiges oder verzweigtes Alkyl mit bis zu 6 Kohlenstoffatomen oder Halogen substituiert sein kann, oder
Cycloalkyl mit 3 bis 7 Kohlenstoffatomen,
oder geradkettiges oder verzweigtes Alkyl mit bis zu 18 Kohlenstoffatomen bedeuten, das gegebenenfalls durch Pyridyl substituiert ist,

$R^7$ geradkettiges oder verzweigtes Alkyl mit bis zu 8 Kohlenstoffatomen bedeutet,

A, B und D gleich oder verschieden sind und
für eine direkte Bindung oder
für einen Rest der Formel

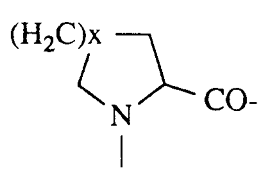

oder

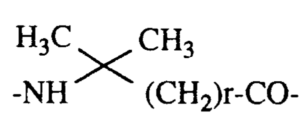

stehen ,
worin

x die Zahl 1 oder 2 bedeutet und

r die Zahl 0 oder 1 bedeutet, oder

für eine Gruppe der Formel

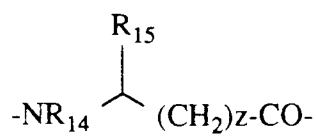

stehen

worin

z die Zahl 0 oder 1 bedeutet,

$R^{14}$ Wasserstoff oder geradkettiges oder verzweigtes Alkyl mit bis zu 4 Kohlenstoffatomen bedeutet,

$R^{15}$ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,

wobei das Alkyl gegebenenfalls durch Cyano, Methylthio, Hydroxy, Mercapto, Guanidyl oder durch eine Gruppe der Formel $-NR^{16}R^{17}$ oder $R^{18}$-OC-substituiert ist,

worin

$R^{16}$ und $R^{17}$ unabhängig voneinander für Wasserstoff, geradkettiges oder verzweigtes Alkyl mit bis zu 8 Kohlenstoffatomen oder Phenyl stehen,

und

$R^{18}$ Hydroxy, Benzyloxy, Alkoxy mit bis zu 6 Kohlenstoffatomen oder die oben aufgeführte Gruppe $-NR^{16}R^{17}$ bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oderdurch die Gruppe $-NR^{16}R^{17}$ substituiert ist,

worin

$R^{16}$ und $R^{17}$ die oben angegebene Bedeutung haben,

oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^{1}$ für geradkettiges oder verzweigtes Alkyl oder Alkenyl mit bis zu 10 Kohlenstoffatomen steht, die gegebenenfalls durch Cycloalkyl mit 3 bis 8 Kohlenstoffatomen oder Aryl mit 6 bis 10 Kohlenstoffatomen substituiert sind, das seinerseits durch Halogen, Nitro, Hydroxy, Amino oder geradkettiges oder verzweigtes Alkoxy mit bis zu 4 Kohlenstoffatomen substituiert sein kann,

E für einen Rest der Formel

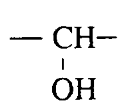 oder 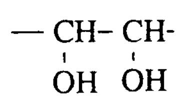

steht,

$R^2$ und $R^3$ gleich oder verschieden sind und für Wasserstoff oder für geradkettiges oder verzweigtes Alkyl mit bis zu 8 Kohlenstoffatomen stehen, oder

$R^2$ und $R^3$ gemeinsam mit dem Stickstoffatom und unter Einbezug der

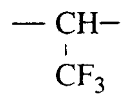

-Gruppe einen 5- bis 7-gliedrigen gesättigten Heterocyclus bilden, an den ein weiterer 5- bis 6-gliedriger, gesättigter, partiell ungesättigter oder aromatischer Carbocyclus ankondensiert sein kann, wobei beide Ringe gegebenenfalls durch geradkettiges oder verzweigtes Alkyl mit bis zu 8 Kohlenstoffatomen substituiert sind

und deren physiologisch unbedenklichen Salze.

**2.** Verbindungen gemäß Anspruch 1

in welcher

W für Wasserstoff, tert.Butyloxycarbonyl (BOC) 9-Fluorenylmethyloxycarbonyl (Fmoc), Benzyloxycarbonyl (Z) oder Pyridylmethoxycarbonyl steht, oder für geradkettiges oder verzweigtes Alkyl oder Alkenyl mit jeweils bis zu 4 Kohlenstoffatomen steht, die gegebenenfalls durch Phenyl substituiert sind, oder für eine Gruppe der Formel $R^4$-CO-, $R^5R^6$N-CO- oder $R^7SO_2$- steht worin

$R^4$ Wasserstoff, Trifluormethyl oder geradkettiges oder verzweigtes Alkoxy mit bis zu 6 Kohlenstoffatomen oder Alkyl mit bis zu 16 Kohlenstoffatomen bedeutet, das gegebenenfalls bis zu 2-fach, gleich oder verschieden, durch Phenyl, Naphthyl oder Pyridyl substituiert sein kann, oder Phenyl oder Naphthyl bedeutet, die gegebenenfalls durch Fluor, Chlor, Trifluormethyl, Trifluormethoxy oder durch geradkettiges oder verzweigtes Alkyl mit bis zu 6 Kohlenstoffatomen substituiert sein können, Cyclopropyl, Cyclopentyl, Cyclohexyl, Chinolyl, Chinolyl-N-oxid, Indolyl, Pyridyl, Morpholino oder Piperazinyl bedeutet, oder einen Rest der Formel

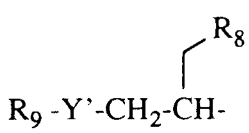

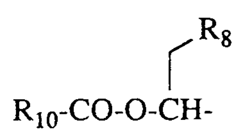

oder

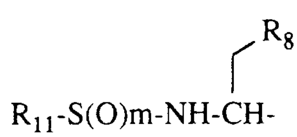

bedeutet,

worin

Y' die CO- oder $SO_2$-Gruppe bedeutet,

$R^8$ Phenyl oder Naphthyl bedeutet,

$R^9$ und $R^{10}$ unabhängig voneinander geradkettiges oder verzweigtes Alkyl mit bis zu 8 Kohlenstoffatomen, Tolyl, Benzyloxy, Phenyl oder Naphthyl bedeuten,

| | |
|---|---|
| $R^{11}$ | die oben angegebene Bedeutung von $R^9$ und $R^{10}$ hat und mit dieser gleich oder verschieden ist oder über N-gebundenes Morpholino oder Pyrrolidinyl bedeutet, |
| m | eine Zähl 1 oder 2 bedeutet, |
| $R^5$ und $R^6$ | unabhängig voneinander Wasserstoff oder<br>Phenyl oder Naphthyl bedeuten, die gegebenenfalls durch geradkettiges oder verzweigtes Alkyl mit bis zu 4 Kohlenstoffatomen, Fluor oder Chlor substituiert sein können,<br>Cyclopropyl, Cyclopentyl oder Cyclohexyl bedeuten, oder<br>geradkettiges oder verzweigtes Alkyl mit bis zu 16 Kohlenstoffatomen bedeuten, das gegebenenfalls durch Pyridyl substituiert ist, |
| $R^7$ | geradkettiges oder verzweigtes Alkyl mit bis zu 6 Kohlenstoffatomen bedeutet, |

A, B und D unabhängig voneinander
für eine direkte Bindung oder
für Prolin stehen, oder
für einen Rest der Formel

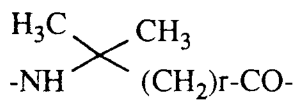

stehen,

worin

| | |
|---|---|
| r | die Zahl 0 oder 1 bedeutet<br>für eine Gruppe der Formel |

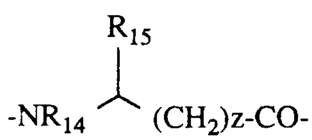

stehen,
worin

| | |
|---|---|
| z | die Zahl 0 oder 1 bedeutet, |
| $R^{14}$ | Wasserstoff, Methyl oder Ethyl bedeutet, |
| $R^{15}$ | Cyclopentyl, Cyclohexyl, Phenyl oder Wasserstoff bedeutet, oder geradkettiges oder verzweigtes Alkyl mit bis zu 6 Kohlenstoffatomen bedeutet,<br>wobei das Alkyl gegebenenfalls durch Cyano, Methylthio, Hydroxy, Mercapto, Guanidyl, Amino, Carboxy oder $H_2N$-CO- substituiert sein kann,<br>oder das Alkyl durch Cyclohexyl, Naphthyl oder Phenyl substituiert ist, das seinerseits durch Fluor, Hydroxy Nitro oder Alkoxy mit bis zu 4 Kohlenstoffatomen substituiert sein kann,<br>oder das Alkyl durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind, |
| $R^1$ | für geradkettiges oder verzweigtes Alkyl oder Alkenyl mit bis zu 8 Kohlenstoffatomen steht, die gegebenenfalls durch Cyclopropyl, Cyclobutyl, Cyclopentyl, Cyclohexyl, Cycloheptyl oder Phenyl substituiert sind, das seinerseits durch Fluor, Chlor, Brom, Nitro, Hydroxy oder Amino substituiert sein kann |
| E | für einen Rest der Formel |

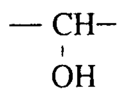 oder 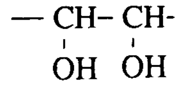

steht,

R² und R³ gleich oder verschieden sind und für Wasserstoff oder für geradkettiges oder verzweigtes Alkyl mit bis zu 6 Kohlenstoffatomen stehen, oder

R² und R³ gemeinsam mit dem Stickstoffatom und unter Einbezug der

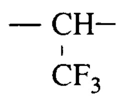

-Gruppe für einen Rest der Formel

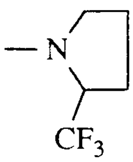 , 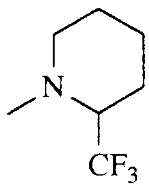 , 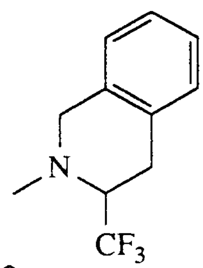 , 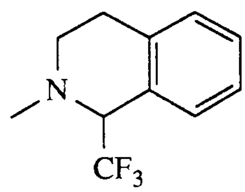

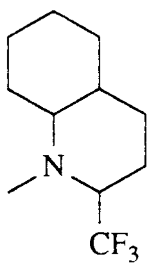 , 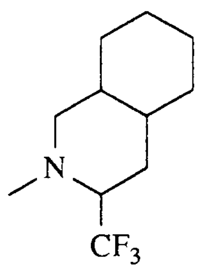 , 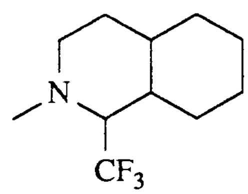

oder 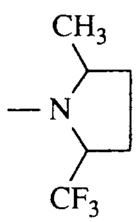

wobei beide Ringe geebenenfalls durch geradkettiges oder verzweigtes Alkyl mit bis zu 6 Kohlenstoffatomen substituiert sind

und deren physiologisch unbedenklichen Salze.

**3.** Verbindungen gemäß Anspruch 1

in welcher

W für Wasserstoff, tert.Butyloxycarbonyl (BOC), Benzyloxycarbonyl (Z) oder Pyridylmethoxycarbonyl steht, oder für Allyl oder Benzyl steht, für eine Gruppe der Formel $R^4$-CO-, $R^5R^6$N-CO- oder $R^7$-$SO_2$- steht,

worin

$R^4$ Wasserstoff oder geradkettiges oder verzweigtes Alkoxy mit bis zu 4 Kohlenstoffatomen, Alkyl mit bis zu 14 Kohlenstoffatomen bedeutet, das gegebenenfalls bis zu 2-fach durch Phenyl, Naphthyl oder Pyridyl substituiert sein kann, oder

Phenyl oder Naphthyl bedeutet, das gegebenenfalls durch Fluor, Chlor, Trifluormethyl, Trifluormethoxy oder durch geradkettiges oder verzweigtes Alkyl mit bis zu 4 Kohlenstoffatomen substituiert sein kann,
Cyclopropyl, Cyclopentyl, Cyclohexyl, Chinolyl, Chinolyl-N-oxid, Indolyl, Pyridyl, Morpholino oder Piperazinyl bedeutet, oder einen Rest der Formel

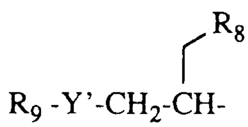

oder

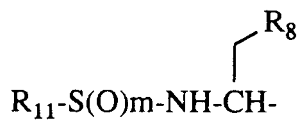

bedeutet,

worin

| | |
|---|---|
| Y' | die CO- oder $SO_2$-Gruppe bedeutet, |
| $R^8$ | Phenyl oder Naphtyl bedeutet |
| $R^9$ | geradkettiges oder verzweigtes Alkyl mit bis zu 4 Kohlenstoffatomen, Tolyl, Benzyloxy, Phenyl oder Naphthyl bedeutet, |
| $R^{11}$ | die oben angegebene Bedeutung von $R^9$ hat und mit dieser gleich oder verschieden ist oder über N-gebundenes Morpholino oder Pyrrolidinyl bedeutet, |
| m | die Zahl 2 bedeutet, |
| $R^5$ und $R^6$ | unabhängig voneinander für Wasserstoff oder Phenyl oder Naphthyl bedeuten, die gegebenenfalls durch Methyl, Fluor oder Chlor substituiert sind, Cyclopropyl, Cyclopentyl oder Cyclohexyl bedeuten, oder geradkettiges oder verzweigtes Alkyl mit bis zu 14 Kohlenstoffatomen bedeuten, das gegebenenfalls durch Pyridyl substituiert ist, |
| $R^7$ | geradkettiges oder verzweigtes Alkyl mit bis zu 4 Kohlenstoffatomen bedeutet, |

A, B und D unabhängig voneinander
für eine direkte Bindung,
Prolin oder
für einen Rest der Formel

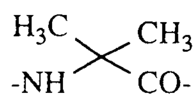

stehen, oder

für eine Gruppe der Formel

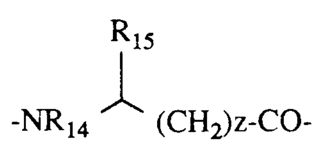

stehen,

worin

| | |
|---|---|
| z | die Zahl 0 oder 1 bedeutet, |
| $R^{14}$ | Wasserstoff oder Methyl bedeutet, |
| $R^{15}$ | Cyclopentyl, Cyclohexyl oder Wasserstoff bedeutet, oder geradkettiges oder verzweigtes Alkyl mit bis zu 4 Kohlenstoffatomen bedeutet, |

wobei das Alkyl gegebenenfalls durch Cyano, Methylthio, Hydroxy, Mercapto, Guanidyl, Amino, Carboxy oder $H_2N$-CO- substituiert sein kann,

oder das Alkyl durch Cyclohexyl, Naphthyl oder Phenyl substituiert ist, das seinerseits durch Fluor, Chlor oder Alkoxy mit bis zu 4 Kohlenstoffatomen substituiert sein kann,

oder das Alkyl durch Indolyl, Imidazolyl, Triazolyl, Pyridyl oder Pyrazolyl substituiert ist, wobei die NH-Funktion gegebenenfalls durch Methyl, Benzyloxymethylen oder tert.Butyloxycarbonyl (BOC) geschützt

$R^1$ für geradkettiges oder verzweigtes Alkyl mit bis zu 6 Kohlenstoffatomen steht, das gegebenenfalls durch Cyclopropyl, Cyclopentyl, Cyclohexyl oder Phenyl substituiert ist, das seinerseits durch Hydroxy substituiert sein kann,

E für einen Rest der Formel

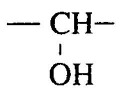 oder 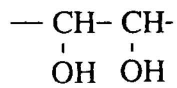

steht,

$R^2$ und $R^3$ gleich oder verschieden sind und für Wasserstoff oder für geradkettiges oder verzweigtes Alkyl mit bis zu 4 Kohlenstoffatomen stehen, oder

$R^2$ und $R^3$ gemeinsam mit dem Stickstoffatom und unter Einbezug der

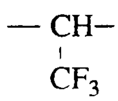

-Gruppe für einen Rest der Formel

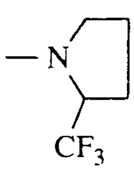 , 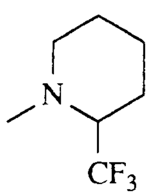 , 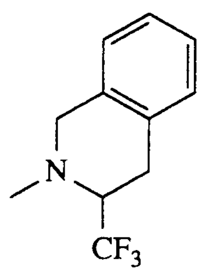 , 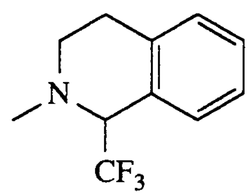 ,

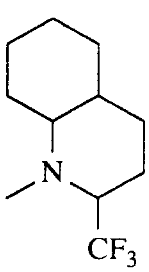 , 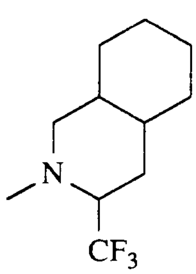 , 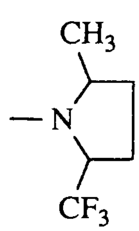 oder 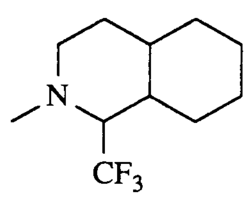

und deren physiologsich unbedenklichen Salze.

4. Verfahren zur Herstellung von Verbindungen der allgemeinen Formel (I) gemäß den Ansprüchen 1-3, dadurch gekennzeichnet, daß man im Fall, daß

E für die

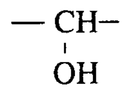

Gruppe steht,

[A] entweder Verbindungen der allgemeinen Formel (III)

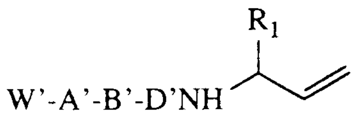

(III)

in welcher

$R^1$ die oben angegebene Bedeutung hat,

A', B' und D' die oben angegebene Bedeutung von A, B und D haben aber nicht gleichzeitig für eine Bindung stehen

und

W' die oben angegebene Bedeutung von W hat, aber nicht für Wasserstoff steht,

zunächst mit einer Epoxidierungsreaktion, gegebenenfalls mit Hilfe einer Base oder einer Phasentransferkatalyse in die Verbindungen der allgemeinen Formel (IV)

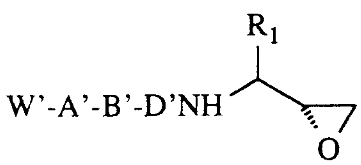

(IV)

in welcher

W', A', B', D' und $R^1$ die oben angegebene Bedeutung haben,

überführt und anschließend mit Verbindungen der allgemeinen Formel (V)

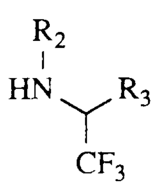

(V)

in welcher

$R^2$ und $R^3$ die oben angegebene Bedeutung haben,

in inerten Lösemitteln umsetzt und gegebenenfalls die Schutzgruppe W' abspaltet (W = H) oder austauscht,

oder

[B] direkt Verbindungen der allgemeinen Formel (IVa)

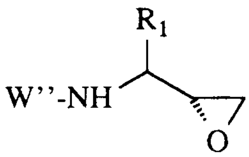

(IVa)

in welcher

$R^1$ die oben angegebene Bedeutung

und

W'' für eine Aminoschutzgruppe, vorzugsweise für Boc oder Z steht,

zunächst durch Umsetzung mit Verbindungen der allgemeinen Formel (V), in die Verbindungen der allgemeinen Formel (VI)

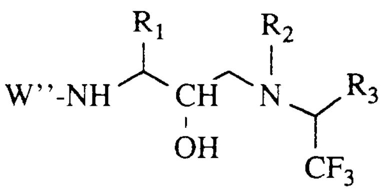

(VI)

in welcher

W'', $R^1$, $R^2$ und $R^3$ die oben angegebene Bedeutung haben,

überführt und anschließend entweder mit Verbindungen der allgemeinen Formel (VII), (VIIa), (VIII) oder (VIIIa)

W'-A-B-D-OH (VII); W'-A'-B'-D'-OH (VIIa);

W-X (VIII); oder $(G)_2O$ (VIIIa)

in welcher

W, W', A, A', B, B', D und D' die oben angegebene Bedeutung haben

X in Abhängigkeit von der Bedeutung des Substituenten W für Hydroxy oder Halogen, vorzugsweise für Chlor steht,

und

G für die $CF_3CO$- oder $H_3C$-CO-Gruppe steht,

im Fall der Verbindungen der allgemeinen Formel (VII) oder (VIIa) nach den in der Peptidchemie üblichen Bedingungen unter Aktivierung der Carbonsäure, gegebenenfalls in Anwesenheit einer Base und eines Hilfsstoffes, und jeweiliger Abspaltung der Schutzgruppen, in einem Schritt (VIIa) oder sukzessive (VII) in inerten Lösemitteln kondensiert, und gegebenenfalls den Substituenten W nach üblichen Methoden variiert,

und im Fall, daß E für die

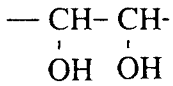

Gruppe steht,
[C] zunächst Verbindungen der allgemeinen Formel (IX)

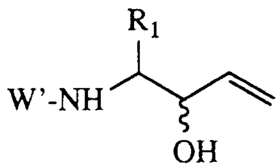 (IX)

in welcher

W' und $R^1$ die oben angegebene Bedeutung haben,

mit der unter [A] beschriebenen Epoxidierungsreaktion in die Verbindungen der allgemeinen Formel (X)

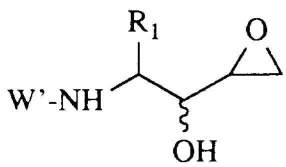 (X)

in welcher

W' und $R^1$ die oben angegebene Bedeutung haben,

überführt, diese mit den Verbindungen der allgemeinen Formel (V) unter Ringöffnung umsetzt und anschließend wie unter [B] beschrieben mit den Verbindungen der allgemeinen Formel (VII), (VIIa), (VIII) und/oder (VIIIa) umsetzt,

und gegebenenfalls eine Trennung der Diastereomere durchführt.

**5.** Arzneimittel enthaltend eine der Verbindungen gemäß den Ansprüchen 1-3.

**6.** Verwendung der Verbindungen gemäß den Ansprüchen 1-3 zur Herstellung von Arzneimitteln.